(19) Europäisches Patentamt
European Patent Office
Office européen des brevets

(11) EP 2 089 518 B1

(12) **EUROPÄISCHE PATENTSCHRIFT**

(45) Veröffentlichungstag und Bekanntmachung des Hinweises auf die Patenterteilung:
**19.11.2014 Patentblatt 2014/47**

(21) Anmeldenummer: **07818346.4**

(22) Anmeldetag: **21.09.2007**

(51) Int Cl.:
***C12N 9/18*** *(2006.01)*

(86) Internationale Anmeldenummer:
**PCT/EP2007/008257**

(87) Internationale Veröffentlichungsnummer:
**WO 2008/040466 (10.04.2008 Gazette 2008/15)**

(54) **KLONIERUNG, EXPRESSION UND VERWENDUNG SAURER PHOSPHOLIPASEN**

CLONING, EXPRESSION AND USE OF ACID PHOSPHOLIPASES

CLONAGE, EXPRESSION ET UTILISATION DE PHOSPHOLIPASES ACIDES

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **02.10.2006 DE 102006046719**

(43) Veröffentlichungstag der Anmeldung:
**19.08.2009 Patentblatt 2009/34**

(73) Patentinhaber: **AB Enzymes GmbH**
**64293 Darmstadt (DE)**

(72) Erfinder:
- **NGUYEN, Khanh Q**
  **64385 Reichelsheim (DE)**
- **MARSCHNER, Volker**
  **64404 Bickenbach (DE)**
- **TITZE, Kornelia**
  **64372 Ober-Ramstadt (DE)**
- **WINTER, Bruno**
  **Ballyphehane, Cork,**
  **Co. Cork (IE)**

(74) Vertreter: **Hiebl, Inge Elisabeth et al**
**Kraus & Weisert**
**Patentanwälte PartGmbB**
**Thomas-Wimmer-Ring 15**
**80539 München (DE)**

(56) Entgegenhaltungen:
**WO-A-03/097825**

- **SHEN D-K ET AL: "Characterisation and expression of phospholipases B from the opportunistic fungus Aspergillus fumigatus" FEMS MICROBIOLOGY LETTERS, AMSTERDAM, NL, Bd. 239, Nr. 1, 1. Oktober 2004 (2004-10-01), Seiten 87-93, XP004579957 ISSN: 0378-1097 in der Anmeldung erwähnt -& DATABASE UniProt [Online] 5. Juli 2004 (2004-07-05), "Lysophospholipase 1 precursor (EC <A HREF="http://srs.ebi.ac.uk/srsbin/cgi-bin/wgetz?[enzyme-ECNumber:3.1.1.5]+-e">3.1.1.5</A>) (Phospholipase B 1)." XP002471088 gefunden im EBI accession no. UNIPROT: Q6U820 Database accession no. Q6U820**
- **DATABASE Geneseq [Online] 4. November 2004 (2004-11-04), "Aspergillus fumigatus essential gene protein #155." XP002471089 gefunden im EBI accession no. GSP:ADR86105 Database accession no. ADR86105**
- **HAKI G D ET AL: "Developments in industrially important thermostable enzymes: A review" BIORESOURCE TECHNOLOGY, ELSEVIER, GB, Bd. 89, Nr. 1, August 2003 (2003-08), Seiten 17-34, XP002463394 ISSN: 0960-8524**
- **HONG SAHYUN ET AL: "Identification and molecular cloning of a gene encoding Phospholipase A2 (plaA) from Aspergillus nidulans." BIOCHIMICA ET BIOPHYSICA ACTA 15 AUG 2005, Bd. 1735, Nr. 3, 15. August 2005 (2005-08-15), Seiten 222-229, XP002471087 ISSN: 0006-3002**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft neue DNA-Sequenzen, die Polypeptide mit Phospholipaseaktivität codieren. Die Erfindung betrifft ferner neue Polypeptide mit Phospholipaseaktivität. Bei diesen Polypeptiden handelt es sich um saure Phospholipasen mit hoher Thermostabilität. Ferner betrifft die Erfindung auch die Verwendung dieser Phospholipasen zur Reduktion phosphorhaltiger Verbindungen, zum Beispiel bei der Herstellung von Speiseölen sowie die Verwendung dieser Phospholipase als Backhilfsmittel, Tierfutterhilfsmittel, Hilfsmittel bei der Textitrohstoffverarbeitung, etc.

Phospholipide, wie Lecithin oder Phosphatidylcholin, bestehen aus Glycerol verestert mit zwei Fettsäuren an der äußeren (sn-1) und an der mittleren (sn-2) Position des Glycerols, sowie einer estergebundenen Phosphatgruppe an der dritten Position (sn-3). Die Phosphatgruppe ihrerseits kann wiederum z.B. mit Aminoalkoholen verestert sein. Phospholipasen katalysieren die Hydrolyse der Acylbindung oder der Esterbindungen der Phospholipide. Bei den Phospholipasen gibt es verschiedene Typen, die sich in ihrem Spaltmuster unterscheiden. Bei den a-cylspaltenden Phospholipasen unterscheidet man die Phospholipasen A1 und A2 die entweder die Acylgruppe an der sn-1 oder der sn-2 Position hydrolysieren und dabei Lysophospholipid produzieren. Aus diesem Grund kann durch Lysophospholipase (LPL) die verbleibende Fettsäure hydrolysiert werden. Für die Lysophospholipasen ist keine Positionsselektivität bekannt.

In der Literatur sind Phospholipasen Typ B beschrieben die z.T. quasi simultan beide Acylgruppen hydrolysieren, ohne dass die Bildung eines Intermediates an Lysolecithin beobachtet werden kann (FEMS Microbiol. Let. 18 (1983) 15-18; Annu. Rev. Biochem. 41 (1972) 129-160). Dies ist oft wie z.B. bei der PLB1 und PLB3 Aktivität von *Saccharomyces cerevisiae* (Biochemistry 1999 May 4; 38(18):5864-5871) dadurch bedingt, dass das Enzym viel mehr LPL-Aktivität als $PLA_n$-Aktivität hat. Reine Lysophospholipasen ohne Phospholipasenebenaktivität können keine Fettsäuren aus Phospholipiden abspalten, die Fettsäuren an den Positionen sn-1 und sn-2 enthalten.

Die vorstehend beschriebenen Phospholipasen des Typs A, von denen die Aminosäure- und/oder Nucleinsäuresequenz bekannt ist, lassen sich in 2 Gruppen einteilen.

a) Die Gruppe der Phospholipasen Typ A mit einem Molekulargewicht von ca. 30-40 kDa. Dazu gehören folgende Phospholipasen aus dem Stand der Technik: In der WO 98/31790 (AB Enzymes GmbH) wird beschrieben, dass in *Aspergillus niger* eine geeignete Phospholipase A zur Entschleimung von Speiseöl gefunden wurde. Das Protein (36 kDa) entfaltet Phospholipaseaktivität nur nach proteolytischer Spaltung, wobei die zwei Fragmente (30 + 6 kDa) über Disulfidbrücken verbunden bleiben. Das Enzym spaltet Lecithin zu Lysolecithin, ist aber auch in der Lage Lysolecithin weiter zu spalten z.B. zum Phosphatidylcholin. In der WO 98/26057 wird eine Phospholipase A aus *Fusarium* sp. beschrieben mit einem Molekulargewicht von 29 ± 10 kDa und einem isoelektrischen Punkt zwischen pl 4.5-8. In der JP 10-155493 A2 wird eine Phospholipase A1 aus *A. oryzae* (295 aa) beschrieben; aus der WO 02/24881 ist eine Phospholipase A aus der Hefe *Zygosascus hellenicus* (407 aa) mit einem isoelektrischen Punkt pl von ca. 4,2 bekannt und in der JP 03151879 wird eine bakterielle Phospholipase aus *Pseudomonas* sp. mit einem Molekulargewicht von ca. 30 kDa beschrieben.
Weiterhin werden in der EP 0 575 133 A2 (US 5,538,874, US 5,378,623, US 5,521,080) Phospholipasen A1 aus *Aspergillen* mit einem Molekulargewicht von 30-40 kDa und einem pl von 2,8-4,5 beschrieben, jedoch ohne Angabe von Sequenzinformationen. Ferner enthalten diese Patentschriften keine Hinweise oder Strategien zum Erhalt der zugehörigen DNA durch Klonierung.

b) Die Gruppe der Phospholipasen Typ A mit einem Molekulargewicht von ca. 60-100 kDa. Dazu gehören: Phospholipasen aus *Hyphozyma,* einer hefeähnlichen Pilzart, beschrieben in WO 98/18912 und Phospholipasen aus *Aspergillus niger,* gemäß WO 03/097825.
Des Weiteren sind in der Literatur Phospholipasen Typ B erwähnt. Bei ihnen handelt es sich um Lysophospholipasen die zusätzlich eine sehr geringe Phospholipase A-Aktivität aufweisen können.

c) Es sind die Sequenzen von mehreren Enzymen mit Molekulargewichten von 45-100 kDa bekannt. Dazu gehören die PLB aus *Aspergillus niger* (WO 01/27251, WO 03/097825), die PLB aus *Aspergillus fumigatus* (Shen et al. FEMS Microbiol Lett. 2004, 239 (1):87-93), die PLB aus *Aspergillus oryzae* (WO 01/27251 & WO 01/29222), die PLB aus *Fusarium venenatum* und *Fusarium verticillioides* (WO 00/28044), die PLB aus *Penicillium notatum* auch *P. chrysogenum* genannt (N. Masuda et al., Eur. J. Biochem., 202: 783-787 (1991)), die PLB 1-3 aus *Saccharomyces cerevisiae* (Lee et al., 1994 J. Biol. Chem. 269: 19725-19730, Merkel et al., 1999 J. Biol. Chem. 274: 28121-28127), die PLB aus *Torulaspora delbrueckii* (alter Name *Saccharomyces rosei*) (Watanabe et al., 1994, FEMS Microbiology Letters 124: 29-34), *Kluy-veromyces lactis* (Oishi et al., 1999 Biosci. Biotechnol. Biochem. 63: 83-90), *Neurospora crassa* (EMBL 042791) und *Schizosaccharomyces pombe* (EMBL O13857).

d) Es sind mehrere Sequenzen von Enzymen mit einem Molekulargewicht von 30-40 kDa bekannt. Dazu gehören die Lysophospholipase aus *Aspergillus foetidus*, EP 0 808 903, die PLB aus A. *niger,* WO 01/27251 und WO 03/097825, die PLB1 und PLB2 aus *Candida albicans* (J. Biol. Chem. 273 (40): 26078-26086, 1998, Medical Mycology 37:61-67, 1998), und die PLB aus *Pseudomonas* PS21, JP 03151879.

Darüber hinaus beschreibt die WO 2004/097012 "Kernpeptide" bekannter Phospholipasen A2 mit einer erhöhten Phospholipaseaktivität.

WO 00/32758, WO 03/060112 und WO 2004/111216 beschreiben Methoden mittels "Protein Engineering" bekannter Lipasen, z.B. aus *Thermomyces lanuginosus* und anderer Phospholipasen zu Enzymvarianten zu gelangen, die eine veränderte Lipase- bzw. Phospholipaseaktivität aufweisen.

Die WO 02/066622 beschreibt neue Gene mit hoher Homologie zu den Genen der *Thermomyces lanuginosus* Lipase sowie deren Verwendung für das gene shuffling zur Erzeugung neuer lipolytischer Enzyme.

DATABASE Geneseq [Online] 4. November 2004 (2004-11-04), "Aspergillus fumigatus essential gene protein #155" offenbart ein "essentielles Protein #155" aus Aspergillus fumigatus, das mit der erfindungsgemäßen SEQ ID NO: 2 in einem 615 aa Überlapp 91,7% Identität enthält.

Phospholipasen werden z.B. zur Entschleimung von Speiseölen verwendet. Dabei werden nicht-hydratisierbare Phospholipide durch Phospholipasen wasserlöslich gemacht und so schonend, kostensparend und umweltfreundlich aus dem Speiseöl entfernt. In dem Patent EP 0 513 709 B2 (Röhm GmbH/Metallgesellschaft AG jetzt AB Enzymes GmbH/mg technologies ag) wird erstmals ein wirksames enzymatisches Verfahren zur Entschleimung vorgestellt. Dabei wird ein mit Wasser vorentschleimtes Speiseöl mit einer wässrigen Lösung einer Phospholipase emulgiert. Nach der Hydrolyse werden die wässrige Phase und die darin enthaltenen Spaltprodukte der phosphorhaltigen Verbindungen abgetrennt. Das enzymatische Entschleimungsverfahren wurde als "EnzyMax-Verfahren" von der Firma Lurgi in der Speiseölindustrie eingeführt. In der DE-A43 39 556 wird als weitere Variante dieses Verfahrens die Wiederverwendung des Enzyms beschrieben, indem man das Enzym aus einer gebrauchten, schlammhaltigen wässrigen Phase durch Zusatz von Tensiden oder Lösungsvermittlern ablöst und als weitgehend schlammfreie, enzymhaltige Lösung wiederverwendet. Lysophospholipasen, Enzyme, die nur Lysolecithin zu spalten vermögen, sind im Entschleimungsprozess wirkungslos.

Phospholipasen werden ferner in der Lebens- und Futtermittelindustrie vielfältig eingesetzt, z.B. bei der Teigherstellung, bei der Herstellung von Backwaren, bei der Herstellung von Milchprodukten etc. Daher werden Phospholipasen benötigt, die sich technologisch vielseitig einsetzen lassen.

Phospholipasen werden auch in der Textilindustrie bei Bioscouring zur Reinigung der Pflanzenfaser vor den weiteren Bearbeitungsschritten wie z.B. dem Einfärben eingesetzt. Hierbei kann auch ein Gemisch aus Phospholipase zusammen mit anderen Enzymen zur Anwendung kommen. Die anderen Enzyme können aus der Gruppe der Cellulasen, Hemicellulasen, Pektinasen, Proteasen, und Oxidoreduktasen ausgewählt sein.

Weitere Einsatzgebiete der Phospholipasen sind die Mayonnaiseherstellung, die Behandlung von Milchprodukten oder deren Verwendung in der Lederbearbeitung (JP-A 7-177884).

Der vorliegenden Erfindung liegt somit die Aufgabe zugrunde, Proteine bzw. Polypeptide mit verbesserten Phospholipaseeigenschaften bereitzustellen. Die neuen Phospholipasen sollen insbesondere keine in technologischen Prozessen relevante Lipaseaktivität aufweisen. Insbesondere sollen die Proteine mit Phospholipaseaktivität eine erhöhte Thermostabilität besitzen.

Ferner sollen die Proteine mit Phospholipaseaktivität einfach, kostengünstig und wirtschaftlich zu produzieren sein. Ferner sollen erfindungsgemäß Expressionskonstrukte bereitgestellt werden, die sich zur Produktion der Proteine mit Phospholipaseaktivität eignen.

Die vorstehenden Aufgaben werden gelöst durch eine DNA-Sequenz, die ein Polypeptid mit Phospholipaseaktivität codiert, dadurch gekennzeichnet, dass die DNA-Sequenz ausgewählt ist aus a) DNA-Sequenzen, die eine Nucleotidsequenz gemäß SEQ ID NO: 1 umfassen, b) DNA-Sequenzen, die die codierende Sequenz gemäß SEQ ID NO: 1 umfassen, c) DNA-Sequenzen, die die Aminosäuresequenz gemäß SEQ ID NO: 2 codieren, d) DNA-Sequenzen, die von dem Plasmid B11B1Hind6 mit der Restriktionskarte gemäß Figur 8 und hinterlegt unter der Hinterlegungsnummer DSM 18369 codiert werden, und g) komplementären Strängen zu den Sequenzen gemäß a) bis d).

Die Erfindung betrifft ferner ein Polypeptid mit Phospholipaseaktivität, ausgewählt aus a) einem Polypeptid, das von dem codierenden Teil einer der vorstehenden DNA-Sequenzen codiert wird, b) einem Polypeptid mit der Sequenz gemäß SEQ ID NO: 2, c) einem Polypeptid mit einer Sequenz, die mindestens 92% Identität zu den Aminosäuren 33 bis 633 von SEQ ID NO: 2 aufweist.

Weiterhin betrifft die Erfindung Expressionskonstrukte bzw. Wirte, die in der Lage sind, die erfindungsgemäßen Polypeptide mit Phospholipaseaktivität zu exprimieren. Ferner betrifft die Erfindung auch die entsprechenden Expressionsplasmide und Vektoren. Weiterhin betrifft die Erfindung Verfahren zur Entschleimung von pflanzlichem Öl unter Verwendung der erfindungsgemäßen Polypeptide sowie die Verwendung der erfindungsgemäßen Polypeptide für Anwendungen im Bereich der Lebensmitteltechnologie, insbesondere zur Herstellung von Teig, Backwaren oder Milchprodukten, bzw. in der Tierernährung und bei der Bearbeitung von Textilrohstoffen, dem sogenannten Scouring oder Bioscouring.

Die im vorstehenden Stand der Technik genannten Sequenzen von Phospholipasen sind vom Schutzumfang der Erfindung expressis verbis ausgenommen. Insbesondere sind vom Schutzumfang der Erfindung die Sequenzen der Proteine mit Phospholipaseaktivität sowie die entsprechenden DNA-Sequenzen der folgenden Dokumente ausgenommen: WO 01/27251, WO 2004/111216, WO 2004/097012, WO 03/060112, WO 02/24881, WO 00/28044, WO 00/32758, WO 03/097825, EP 999 73 065.8 sowie dazugehörige Teilanmeldungen. Die Ausnahme der Sequenzen betrifft diese Druckschriften in ihrer Gesamtheit sowie einzeln und in beliebigen Kombinationen.

Überraschenderweise wurde gefunden, dass eine DNA-Sequenz, die ein Polypeptid mit Phospholipaseaktivität codiert, das ein hohes Molekulargewicht sowie eine erhöhte Thermostabilität besitzt, aus einem Stamm der Gattung *Aspergillus fumigatus* isoliert werden kann. Bei dieser Phospholipase handelt es sich um eine saure, von einem filamentösen Pilz abstammende Phospholipase mit einem berechneten Molekulargewicht von 65 kDa, die in der Lage ist, mindestens eine der zwei Fettsäuren aus Lecithin zu hydrolysieren. Dieses Enzym zeichnet sich weiterhin dadurch aus, dass das Protein unter denaturierenden Bedingungen in zwei Fragemente gespalten werden kann. Dabei tritt ein Fragment mit 34 kDa, bzw. nach Deglycosilierung mit N-Glycosidase F von 20 kDa und ein Fragment von circa 72 kDa (nach Deglycosilierung mit N-Glycosidase F von 54 kDa) auf. Diese Fragmente tragen außer der N-Glycosilierung auch O-Glycosilierungen wie der Vergleich mit dem aus der Aminosäuresequenz abgeleiteten Molekulargewicht zeigt (18,4 und 46,6 kDa).

Diese Phospholipase besitzt unter den Bedingungen der enzymatischen Entschleimung von Speiseöl keine für diesen Prozess relevante Lipaseaktivität und kann so in einem Verfahren zur enzymatischen Entschleimung von Speiseölen vorteilhafterweise eingesetzt werden, da sie im Öl keine oder nur nicht nennenswerte Anteile an Triglyceridbindungen hydrolysiert. Ferner besitzt die erfindungsgemäße Phospholipase im Gegensatz zu Phospholipasen aus dem Stand der Technik (zum Beispiel WO 98/31790) bereits ohne proteolytische Spaltung die volle Phospholipaseaktivität. Im Gegensatz zu den Polypeptiden mit Phospholipaseaktivität, die aus dem Stand der Technik bekannt sind, verfügen die erfindungsgemäßen Phospholipasen über eine erhöhte Thermostabilität und können dadurch vorteilhafterweise auch in Prozessen der enzymatischen Entschleimung bei höheren Temperaturen eingesetzt werden. Dies ist vor allem ökonomisch interessant, da somit bei den Entschleimungsprozessen nicht zuerst die Temperatur des Öls erniedrigt werden muss, um die enzymatische Entschleimung ohne Inaktivierung des Enzyms zu ermöglichen und anschließend die Temperatur des Öls erhöht werden muss, um die Viskosität des Öls für den Zentrifugationsschritt zur Trennung der Öl- und Wasserphasen zu erniedrigen. Auch für andere Anwendungen im Bereich der Lebensmitteltechnologie und Tierernährung bzw. in der Textilverarbeitung ist die erhöhte Thermostabilität der erfindungsgemäßen Polypeptide mit Phospholipaseaktivität vorteilhaft.

Phospholipasen mit hohem Molekulargewicht von filamentösen Pilzen, die durch die vorstehend zitierten Sequenzen codiert sind, haben Temperaturoptima von 25° bis 55°C. Das vorliegende erfindungsgemäße Enzym zeigt auch bei einer Anwendung bei 65°C über 6 h Aktivität. Auch eine wiederholte Benutzung des Enzyms über mehrere Entschleimungszyklen ist bei höheren Temperaturen möglich.

Die erhöhte Thermostabilität der erfindungsgemäßen Phospholipase war überraschend und aufgrund der im Stand der Technik beschriebenen Phospholipasen nicht naheliegend. Zu keiner der im Stand der Technik beschriebenen natürlich vorkommenden Phospholipasen aus filamentösen Pilzen sind diese Eigenschaften beschrieben oder auch nur nahegelegt.

Da es keine Veröffentlichung zu thermostabilen Phospholipasen aus filamentösen Pilzen gibt und damit auch keine Anhaltspunkte weiche Strukturelemente (Helices, β-Faltblätter, Loops) bei einer Phospholipase speziell gestaltet sein müssen (Vorliegen von ionischen Wechselwirkungen über geladene Aminosäuren und wenn möglich polyvalente Ionen, Disulfidbrücken, van-der-Waals Wechselwirkungen, hydrophobe Wechselwirkungen), um eine hohe Thermostabilität zu gewährleisten, war es nicht vorherzusehen, dass die gefundene Phospholipase diese Eigenschaften besitzen würde.

Die Gene der Familie der "GX"-Lipasen im weitesten Sinne, zu denen auch die Phospholipasen und Lysophospholipasen gehören, zeigen, dass kleine Veränderungen an der Sequenz die Eigenschaften des von dieser Sequenz codierten Enzyms stark beeinflussen. Dies ist z.B. in der Anmeldung WO 03/060112 gezeigt. Diese Anmeldung beschreibt ein Verfahren zur Erzeugung von Varianten von lipolytischen Enzymen. Dabei werden Änderungen an der Substratspezifität über random-Mutagenese erreicht, nicht über spezifische, gerichtete, Mutationen. Diese Anmeldung zeigt auch, dass trotz hoher Homologie in der Sequenz die Eigenschaft davon codierten Enzyms nicht vorhergesagt werden kann. Diese mangelnde Korrelation zwischen DNA-Sequenz und codiertem Enzym sowie Unterschiede in der Codonusage durch die einzelnen Stämme erlauben keine Ableitung von Primern aus bekannten Sequenzen zum Auffinden von neuen Sequenzen mit gezielten Eigenschaften, wie hoher Thermostabilität und geringer Lipaseaktivität.

Gemäß einer weiteren Ausführungsform betrifft die Erfindung ein Polypeptid mit Phospholipaseaktivität, das dadurch gekennzeichnet ist, dass es ein Molekulargewicht im Bereich von 63 bis 76 kDa besitzt und auch als Fragmente von 18,4 kDa und 46,6 kDa (unglykosiliert) auftreten kann, mindestens eine der zwei Fettsäuren aus Lecithin hydrolysieren kann, keine Lipaseaktivität aufweist, eine erhöhte Thermostabilität besitzt und aus einem Organismus der Gattung *Aspergillus* isolierbar ist.

Dabei ist unter erhöhter Thermostabilität zu verstehen, dass das Enzym bei einer Temperatur von 65°C über mindestens 6 h eine Aktivität von mindestens 80% beibehält unter den Bedingungen bei der Ölentschleimung mit geringem Wasseranteil von 1 - 5%.

Die erfindungsgemäße Phospholipasesequenz gemäß SEQ ID NO: 2 wurde mit Phospholipasesequenzen aus dem Stand der Technik verglichen. Eine hohe Übereinstimmung auf der Ebene der Aminosäuresequenz wurde zu der aus WO 03/097825 bekannten Aminosäuresequenz aus *Aspergillus niger* gefunden, d.h. die Übereinstimmung betrug 74%.

Die erfindungsgemäße Phospholipasesequenz gemäß SEQ ID NO: 1 wurde mit Lysophospholipasesequenzen aus dem Stand der Technik verglichen, da diese z.T. sehr geringe Phospholipaseaktivitäten aufweisen können, es sich jedoch definitionsgemäß um Lysophospholipasen handelt. Die Sequenz SEQ ID NO: 1 zeigt die höchste Identität von 91% mit der Sequenz aus (Shen et al., FEMS Microbiol. Letters 2004, 239 (1): 87-93) mit der Accession Number AAQ85122 und 76% 1-dentität mit der Sequenz 8 aus US 6,759,225 (WO 01/027251), die aus *Aspergillus oryzae* stammt. Trotz der hohen Übereinstimmung in der Aminosäuresequenz handelt es sich um verschiedene Enzyme. Das erfindungsgemäße Enzym hat eine überwiegende Phospholipaseaktivität, die Enzyme aus Shen et al. 2004 und US 6,759,225 werden als Lysophospholipase bezeichnet. Enzymaktivitätsdaten zu deren potentieller Phospholipaseaktivität liegen in den genannten Veröffentlichungen nicht vor.

Die Erfindung betrifft somit auch Polypeptide mit Phospholipaseaktivität mit einer Sequenz, die mindestens 92% Identität zu der Sequenz gemäß SEQ ID NO: 1 besitzt. Bevorzugt betrifft die Erfindung ein Polypeptid mit Phospholipaseaktivität mit einer Sequenz, die mindestens 92% Identität zu den Aminosäuren 33 bis 633 von SEQ ID NO: 1 besitzt. Bevorzugt beträgt der Identitätsgrad zu den Aminosäuren 33 bis 633 von SEQ ID NO: 1 mindestens 95%, bevorzugter mindestens 97% und besonders bevorzugt mindestens 98%, unter der Bedingung, dass die jeweiligen Sequenzen Phospholipaseaktivität aufweisen.

Der Grad der Sequenzidentität wird dabei bevorzugt so bestimmt, dass man die Anzahl der Reste der kürzeren Sequenz, die an dem Vergleich beteiligt ist und die ein "entsprechendes" Gegenstück in der anderen Sequenz besitzt, ermittelt. Für die Zwecke der vorliegenden Erfindung wird die Identität dabei bevorzugt in üblicher Weise unter Verwendung der üblichen Algorithmen bestimmt. Erfindungsgemäß werden zum Vergleich nur die cDNAs bzw. Aminosäuren der jeweiligen reifen Proteine herangezogen. Als homologe Sequenzen wurden erfindungsgemäß ähnliche, bevorzugt identische, Sequenzgegenstücke mit Hilfe von bekannten Computerprogrammen ermittelt. Ein Beispiel für ein derartiges Programm ist das Programm Clone Manager Suite, das den Programmteil Align Plus enthält und von Scientific & Educational Software, Durham, NC, USA vertrieben wird. Dabei wird ein Vergleich zweier wie vorstehend definierter DNA- bzw. Aminosäuresequenzen durchgeführt, unter der Option local alignment entweder nach der Methode FastScan - MaxScore oder nach der Methode Needleman-Wunsch unter Beibehaltung der Defaultwerte. Speziell wurde erfindungsgemäß zur Berechnung der Identität die Programmversion "Clone Manager 7 Align Plus 5" mit den Funktionen "Compare Two Sequences/Local Fast Scan-Max Score/Compare DNA sequences", bzw. für Aminosäuren "Compare Two Sequences/Global/Compare sequences as Amino Acids" angewendet. Dabei wurden die aus den folgenden Quellen zugänglichen Algorithmen verwendet: Hirschberg, D.S. 1975. A linear space algorithm for computing longest common subsequences. Commun Assoc Comput Mach 18:341-343; Myers, E.W. and W. Miller. 1988. Optimal alignments in linear space. CABIOS 4:1, 11-17; Chao, K-M, W.R. Pearson and W. Miller. 1992. Aligning two sequences within a specified diagonal band. CABIOS 8:5, 481-487.

Beschrieben werden ferner Additions- und/oder Deletionsmoleküle der vorstehenden Polypeptide mit Phospholipaseaktivität. So kann ein modifiziertes Polypeptid mit Phospholipaseaktivität durch Anfügen weiterer Sequenzen am N-terminalen und/oder C-terminalen Ende verlängert werden, wobei die so erhaltenen Aminosäuresequenzen noch Phospholipaseaktivität aufweisen müssen. Dadurch können Hybridmoleküle hergestellt werden, die weitere vorteilhafte Eigenschaften besitzen. Beispielsweise können Suspensionsproteine bzw. deren native Vorläuferformen in hohem Maße sekretierte Proteine angefügt werden, wodurch die Sekretionseffizienz weiter verbessert wird. Ferner können aktive Sequenzabschnitte anderer Enzyme hinzugefügt werden, um Enzyme mit mehrfacher Spezifität herzustellen. Ferner können polare oder unpolare Sequenzen angefügt werden, um die Löslichkeitseigenschaften bzw. die Membrangängigkeit des so erhaltenen Enzyms in gezielter Weise zu beeinflussen.

Es können auch Sequenzabschnitte des Polypeptids mit Phospholipaseaktivität deletiert werden unter Beibehaltung der Phospholipaseaktivität. Die Mutationen, Elongationen und Verkürzungen können in an sich bekannter Weise nach auf dem Fachgebiet gut bekannten Verfahren durchgeführt werden. Verkürzte Polypeptide zeichnen sich häufig durch eine verbesserte Sekretionshöhe im Vergleich zu den Volllängenpolypeptiden aus. Sie können auch höhere Thermostabilitäten im Vergleich zum Volllängenpolypeptid aufweisen, da sie nur den "komprimierten Kern" enthalten.

Die Erzeugung solcher Varianten ist allgemein auf dem Fachgebiet bekannt. Beispielsweise können Aminosäuresequenz-Varianten der Polypeptide durch Mutation in der DNA hergestellt werden. Verfahren zur Mutagenese und Nucleotidsequenz-Veränderung sind auf dem Fachgebiet gut bekannt (vgl. beispielsweise Kunkel, Proc. Natl. Acad. Sci. USA, 82:488 (1985), Kunkel et al., Methods in Enzymol., 154:367 (1987), US-Patent Nr. 4,873,192, Walker und Gaastra, eds., Techniques in Molecular Biology, Mac Millan Publishing Company, New York (1983)). Hinweise über geeignete Aminosäuresubstitutionen, die die biologische Aktivität des Proteins von Interesse nicht beeinträchtigen, finden sich in dem Model von Dayhoff et al., Atlas of Protein Sequence and Structure, Natl. Biomed. Res. Found., Washington, D.C. (1978). Konservative Substitutionen wie der Austausch einer Aminosäure gegen eine andere mit ähnlichen Eigenschaften sind bevorzugt. Diese Austausche lassen sich in zwei Hauptgruppen mit zusammen vier Untergruppen einteilen und ein Austausch in jeder Untergruppe wird als konservativer Austausch bezeichnet, der bevorzugt nicht die Aktivität oder die Faltung des Proteins beeinflusst.

| | | |
|---|---|---|
| Aliphatisch | Nicht-Polar | G A P |
| | | I L V |
| | Polar und ungeladen | C S T M N Q |
| | Polar und geladen | D E |
| | | K R |
| Aromatisch | | H F W Y |

Die Ausdrücke "Protein", "Peptid" und "Polypeptid" werden im Wesentlichen austauschbar verwendet. Ein Polypeptid oder Enzym mit Phospholipaseaktivität oder eine Phospholipase soll ein Enzym bezeichnen, das die Freisetzung von Fettsäuren aus Phospholipiden, zum Beispiel Lecithinen, katalysiert. Die Phospholipaseaktivität kann unter Verwendung an sich bekannter, beliebiger Assays, bei denen eines dieser Substrate verwendet wird, bestimmt werden.

Im Zusammenhang mit den erfindungsgemäßen Polypeptiden soll der Ausdruck "Phospholipase" bzw. Phospholipase A sowohl Enzyme mit Phospholipase A1- als auch Phospholipase A2-Aktivität bezeichnen. Dabei sind Phospholipase A1 bzw. A2 definiert gemäß der Standardenzym-EC-Klassifikation als EC 3.1.1.2 bzw. 3.1.1.4.

Phospholipase B oder Lysophospholipase sind Polypeptide gemäß der Standardenzym-EC-Klassifikation EC 3.1.1.5.

Die Erfindung betrifft ferner DNA-Sequenzen, die ein Polypeptid mit Phospholipaseaktivität codieren, die Mutationen, Modifikationen bzw. Variationen der Sequenz gemäß SEQ ID NO: 1 umfassen. Beschrieben sind auch Sequenzen, die unter relaxierten oder stringenten Bedingungen mit den vorstehenden Sequenzen hybridisieren. Als stringente Bedingungen gelten: Hybridisierung bei 65°C, 18 h in Dextransulfat-Lösung (GenescreenPlus, DuPont), danach waschen der Filter jeweils 30 min zuerst mit 6 x SSC, zweimal 2 x SSC, zweimal 2 x SSC, 0,1% SDS und anschließend mit 0, 2 x SSC bei 65°C (Membrane transfer and detection methods, Amersham).

Ferner betrifft die Erfindung auch DNA-Sequenzen, die aufgrund der Degeneriertheit des genetische Codes mit den vorstehenden erfindungsgemäßen Sequenzen verwandt sind. Die Degeneriertheit des genetischen Codes kann sich dabei aufgrund natürlicher Degeneriertheit oder aufgrund eines speziell gewählten Codongebrauchs ergeben. Natürlich vorkommende allelische Varianten können unter Verwendung gut bekannter Techniken der Molekularbiologie, wie zum Beispiel der Polymerasekettenreaktion (PCR) und Hybridisierungstechniken, identifiziert werden.

Die Erfindung betrifft ferner ein Verfahren zur Herstellung eines Polypeptids mit Phospholipaseaktivität nach rekombinanten Techniken umfassend die Züchtung rekombinanter prokaryotischer und/oder eukaryotischer Wirtszellen, die eine erfindungsgemäße DNA-Sequenz enthalten unter Bedingungen, die die Expression des Enzyms fördern, sowie die anschließende Gewinnung des Enzyms. Die Erfindung betrifft ferner die Verwendung der erfindungsgemäßen Polynucleotidsequenzen zur Herstellung von Sonden zum Auffinden ähnlicher Sequenzen, die entsprechende Enzyme codieren, in anderen Organismen sowie zur Transformation von Wirtszellen.

Eine DNA-Sequenz, die ein erfindungsgemäßes Polypeptid codiert, kann verwendet werden, um beliebige Wirtszellen, wie beispielsweise Zellen von Pilzen, Hefen, Bakterien, Pflanzen oder Säugern zu transformieren. Derart transformierte Zellen zeichnen sich durch eine Sekretion der erfindungsgemäßen Phospholipase aus. Das so produzierte Phospholipaseenzym bewirkt eine effiziente Hydrolyse der Fettsäuren aus Phospholipiden.

Die Erfindung betrifft auch Expressionskassetten, die zur Einschleusung der eine erfindungsgemäße Phospholipase codierenden DNA-Sequenz bzw. eines offenen Leserasters in eine Wirtszelle verwendet werden können. Sie umfassen bevorzugt eine Transkriptionsstartregion, die mit dem offenen Leseraster verknüpft ist. Eine derartige Expressionskassette kann eine Vielzahl von Restriktionsspaltstellen zur Insertion des offenen Leserasters und/oder anderer DNAs, z.B. einer Transkriptions-Regulator-Region und/oder selektierbare Markergene enthalten. Die Transkriptionskassette umfasst in der 5'→3'-Richtung der Transkription eine Transkriptions- und Translationsstartregion, die DNA-Sequenz von Interesse und eine Transkriptions- und Translationsstopregion, die in einer mikrobiellen Zelle funktionell ist. Die Terminationsregion kann bezüglich der Transkriptioninitiationsregion nativ sein, kann bezüglich der DNA-Sequenz von Interesse nativ sein oder kann von einer beliebigen anderen Quelle abgeleitet sein.

Der Ausdruck "offenes Leseraster" (ORF) bezeichnet die Aminosäure-Sequenz, die zwischen den Translationsstart- und -Stop-Codons einer codierenden Sequenz codiert wird. Die Ausdrücke "Start-Codon" und "Stop-Codon" bezeichnen eine Einheit aus drei benachbarten Nucleotiden (Codons) in einer codierenden Sequenz, die den Kettenstart und -stop der Proteinsynthese (mRNA-Translation) spezifizieren.

"Funktionelle Verknüpfung" bezeichnet im Zusammenhang mit einer Nucleinsäure eine Verbindung als Teil des gleichen Nucleinsäuremoleküls in geeigneter Positionierung und Orientierung zum Transkriptionsstart des Promotors. DNA in funktioneller Verknüpfung an einen Promotor befindet sich unter der Transkriptions-Initiations-Regulation des Promotors. Codierende Sequenzen können funktionell mit der Regulatorsequenz in Sense- oder Antisense-Orientierung verknüpft sein. Bei Bezugnahme auf Polypeptide bedeutet funktionelle Verknüpfung die Verbindung als Teil desselben Polypeptids, d.h. über Peptidylbindungen.

Erfindungsgemäß können beliebige Promotoren verwendet werden. Promotor bezeichnet die Nucleotidsequenz üblicherweise stromaufwärts (5') bezüglich der codierenden Sequenz und kontrolliert die Expression der codierenden Sequenz, indem die Erkennung für die RNA-Polymerase und anderer Faktoren, die für die richtige Transkription erforderlich sind, bereitgestellt wird. Der erfindungsgemäß verwendete Promotor kann einen Minimalpromotor umfassen, d.h. eine kurze DNA-Sequenz aus einer TATA-Box und anderen Sequenzen, die die Transkriptionsstartstelle spezifizieren, an die Regulatorelemente zur Kontrolle der Expression angefügt sind.

Der erfindungsgemäß verwendete Promotor kann auch eine Nucleotidsequenz umfassen, die einen Minimalpromotor und Regulatorelemente umfasst, der die Expression einer codierenden Sequenz oder funktionellen RNA kontrollieren kann. Dieser Typ von Promotorsequenz besteht aus proximalen und distalen stromaufwärts gelegenen Elementen, wobei die zuletzt genannten Elemente oft als Enhancer bezeichnet werden. Folglich ist ein Enhancer eine DNA-Sequenz, die die Promotor-Aktivität stimulieren kann und kann ein dem Promotor innewohnendes Element oder ein insertiertes heterologes Element sein, um die Expressionshöhe oder Gewebsspezifität eines Promotors zu verstärken. Er kann in beiden Orientierungen funktionieren und kann selbst bei stromaufwärtiger oder stromabwärtiger Platzierung von dem Promotor funktionieren. Sowohl Enhancer als auch andere stromaufwärts gelegene Promotor-Elemente binden sequenzspezifisch DNAbindende Proteine, die ihre Wirkungen vermitteln. Promotoren können in ihrer Gesamtheit von einem nativen Gen abgeleitet sein oder können aus verschiedenen Elementen zusammengesetzt sein, die von verschiedenen natürlich vorkommenden Promotoren abgeleitet sind oder können sogar aus synthetischen DNA-Segmenten zusammengesetzt sein. Ein Promotor kann auch DNA-Sequenzen enthalten, die an der Bindung von Proteinfaktoren beteiligt sind, die die Effizienz der Transkriptionsinitiation als Antwort auf physiologische oder entwicklungsbedingte Bedingungen kontrollieren.

Promotorelemente, insbesondere TATA-Elemente, die inaktiv sind oder eine stark verminderte Promotor-Aktivität in Abwesenheit einer stromaufwärtigen Aktivierung besitzen, werden als Minimalpromotoren oder Kernpromotoren bezeichnet. In Gegenwart eines geeigneten Transkriptionfaktors bzw. geeigneter Transkriptionsfaktoren liegt die Funktion des Minimalpromotors in der Ermöglichung der Transkription. Ein Minimal- oder Kernpromotor besteht somit nur aus allen Grundelementen, die für die Transkriptionsinitiation notwendig sind, z. B. einer TATA-Box und/oder einem Initiator.

Die Erfindung betrifft auch erfindungsgemäße DNA-Sequenzen enthaltende Vektorkonstrukte. Diese Vektorkonstrukte umfassen beliebige Plasmide, Cosmide, Phagen und andere Vektoren in doppelsträngiger oder einzelsträngiger, linearer oder zirkulärer Form, die gegebenenfalls selbst transmittierbar oder mobilisierbar sein können und die einen prokaryotischen oder eukaryotischen Wirt entweder durch Integration in das zelluläre Genom transformieren können oder die extrachromosomal vorliegen (z. B. autonom replizierende Plasmide mit einem Replikationsorigin).

Vektoren, Plasmide, Cosmide, künstliche Hefechromosomen (YACs), künstliche Bakterienchromosomen (BACs) und DNA-Segmente zur Verwendung zur Transformation von Zellen umfassen allgemein die die erfindungsgemäße Phospholipase codierende DNA sowie eine andere DNA, wie cDNA, ein Gen oder Gene, die in die Zellen eingeschleust werden sollen. Diese DNA-Konstrukte können weitere Strukturen wie Promotoren, Enhancer, Polylinker oder auch Regulatorgene, so weit erforderlich, umfassen. Eines der DNA-Segmente oder Gene, das bzw. die für die zelluläre Einschleusung ausgewählt wurde bzw. wurden, codiert/codieren zweckdienlicherweise ein Protein, das in den so erhaltenen transformierten (rekombinanten Zellen) exprimiert wird, was zu einem screenbaren oder selektierbaren Merkmal führt und/oder der transformierten Zelle einen verbesserten Phenotyp verleiht.

Die Konstruktion von Vektoren, die erfindungsgemäß verwendet werden können, ist einem Fachmann auf dem Gebiet angesichts der vorstehenden Offenbarung und des allgemeinen Fachwissens bekannt (vgl. z. B. Sambrook et al., Molecular Cloning: A Laboratory Manual (2. Aufl., Cold Spring Harbor Laboratory Press, Plainview, N.Y. (1989)).

Eine erfindungsgemäße Expressionskassette kann eine oder mehrere Restriktionsschnittstelle(n) enthalten, um das die Phospholipase codierende Polynucleotid unter die Regulation einer Regulatorsequenz zu stellen. Die Expressionskassette kann auch ein Terminationssignal in funktioneller Verknüpfung mit dem Polynucleotid sowie Regulatorsequenzen, die für die ordnungsgemäße Translation des Polynucleotids benötigt werden, enthalten. Die Expressionskassette, die das erfindungsgemäße Polynucleotid enthält, kann chimär sein, d.h. mindestens eine ihrer Komponenten ist bezogen auf mindestens eine der anderen Komponenten heterolog. Die Expression des Polynucleotids in der Expressionskassette kann unter der Kontrolle eines konstitutiven Promotors, eines induzierbaren Promotors, eines regulierten Promotors, eines viralen Promotors oder eines synthetischen Promotors stehen.

Die Vektoren können bereits Regulatorelemente enthalten, z.B. Promotoren, oder die erfindungsgemäßen DNA-Sequenzen können so manipuliert werden, dass sie solche Elemente enthalten. Geeignete Promotorelemente, die verwendet werden können, sind auf dem Fachgebiet bekannt und sind beispielsweise für *Trichoderma reesei* der cbh1- oder der cbh2-Promotor, für *Aspergillus oryzae* der amy-Promotor, für *Aspergillus niger* der xyl-, glaA-, alcA-, aphA-, tpiA-, gpdA-, sucl- und der pkiA-Promotor. Geeignete Promotorelemente, die zur Expression in Hefe verwendet werden können, sind auf dem Fachgebiet bekannt und sind beispielsweise der pho5-Promotor oder der gap-Promotor zur Expression in *Saccharomyces cerevisiae* und für *Pichia pastoris,* z.B. der aoxl-Promotor oder der fmd-Promotor oder der mox-Promotor für *H. polymorpha.*

DNA, die zur Einschleusung in Zellen geeignet ist, kann neben der erfindungsgemäßen DNA auch DNA umfassen, die aus einer beliebigen Quelle abgeleitet wurde oder daraus isoliert ist. Ein Beispiel für eine abgeleitete DNA ist eine DNA-Sequenz, die als nützliches Fragment in einem gegebenen Organismus identifiziert wurde und die dann in im Wesentlichen reiner Form chemisch synthetisiert wurde. Ein Beispiel für eine solche DNA ist eine geeignete DNA-Sequenz, die beispielsweise unter Verwendung von Restriktionsendonucleasen erhalten wurde, so dass sie weiter erfindungsgemäß manipuliert, beispielsweise amplifiziert werden kann. Zu diesen zählt unter anderem das als Markergen verwendbare amdS-Gen aus *Aspergillus nidulans* und seine regulatorische Sequenzen, sowie Polylinker.

Eine derartige DNA wird üblicherweise als rekombinante DNA bezeichnet. Daher umfasst eine geeignete DNA vollständig synthetische DNA, semisynthetische DNA, aus biologischen Quellen isolierte DNA und von eingeschleuster RNA abgeleitete DNA. Im Allgemeinen ist die eingeschleuste DNA kein ursprünglicher Bestandteil des Genotyps der Empfänger-DNA, aber erfindungsgemäß kann auch ein Gen aus einem gegebenen Genotyp isoliert und eventuell verändert werden und anschließend können Mehrfachkopien des Gens in den gleichen Genotyp eingeschleust werden, z. B. um die Produktion eines gegebenen Genprodukts zu verstärken.

Die eingeschleuste DNA umfasst ohne Beschränkung DNA aus Genen, wie beispielsweise aus Bakterien, Hefen, Pilzen oder Viren. Die eingeschleuste DNA kann modifizierte oder synthetische Gene, Teile von Genen oder chimäre Gene einschließlich Genen aus dem gleichen oder einem unterschiedlichen Genotyp umfassen. Hierzu kann z.B. auch DNA der Plasmide pUC18, pUC19 gehören.

Die zur Transformation erfindungsgemäß verwendete DNA kann zirkulär oder linear, doppelsträngig oder einzelsträngig sein. Im Allgemeinen liegt die DNA in Form einer chimären DNA wie eine Plasmid-DNA vor, die auch codierende Regionen enthält, die von Regulatorsequenzen flankiert sind und die Expression der in der transformierten Zelle vorhandenen rekombinanten DNA unterstützen. Beispielsweise kann die DNA selbst einen Promotor enthalten oder daraus bestehen, der in einer Zelle aktiv ist, der von einer Quelle, die sich von der Zelle unterscheidet, abgeleitet ist oder es kann ein Promotor verwendet werden, der bereits in der Zelle, d.h. der Transformationszielzelle, vorhanden ist.

Im Allgemeinen ist die eingeschleuste DNA relativ klein, weniger als etwa 30 kb, um die Empfindlichkeit gegenüber physikalischem, chemischem oder enzymatischem Abbau zu minimieren, der mit der Größe der DNA zunimmt.

Die Selektion eines geeigneten Expressionsvektors hängt von den Wirtszellen ab. Hefe- oder Pilzexpressionsvektoren können einen Replikationsorigin, einen geeigneten Promotor und Enhancer umfassen, und auch beliebige notwendige Ribosomenbindungsstellen, Polyadenylierungsstellen, Splice-Donor und -Akzeptor-Stellen, Transkriptionsterminationssequenzen und nicht-transkribierte 5'-flankierende Sequenzen umfassen.

Beispiele für geeignete Wirtszellen sind: Pilzzellen der Gattung *Aspergillus, Rhizopus, Trichoderma, Neurospora, Mucor, Penicillium,* etc., wie beispielsweise Hefen der Gattungen *Kluyveromyces, Saccharomyces, Schizosaccharomyces, Trichosporon, Schwanniomyces, Hansenula, Pichia* und dergleichen. Geeignete Wirtssysteme sind beispielsweise Pilze wie *Aspergilli,* z.B. *Aspergillus niger* (ATCC 9142) oder *Aspergillus ficuum* (NRLL 3135) oder *Trichoderma* (z.B. *Trichoderma reesei* QM6a) und Hefen wie *Saccharomyces,* z.B. *Saccharomyces cerevisiae* oder *Pichia,* wie z.B. *Pichia pastoris* oder *Hansenula,* z.B. *H. polymorpha* (DSMZ 70277). Derartige Mikroorganismen können von anerkannten Hinterlegungsstellen, z.B. der American Type Culture Collection (ATCC), dem Centraalbureau voor Schimmelcultures (CBS) oder der Deutschen Sammlung für Mikroorganismen und Zellkulturen GmbH (DSMZ) oder beliebigen anderen Hinterlegungsstellen erhalten werden.

Die Expressionskassette kann in der 5'-3'-Transkriptionsrichtung eine Transkriptions- und Translationstartregion des erfindungsgemäßen Polynucleotids und eine Transkriptions- und Terminationsregion, die in vivo oder in vitro funktionell ist, enthalten. Die Terminationsregion kann bezüglich der Transkriptionsinitiationsregion nativ sein oder kann bezüglich des Polynucleotids nativ oder anderer Herkunft sein. Die Regulatorsequenzen können stromaufwärts (5' nicht-codierende Sequenzen), innerhalb (Intron) oder stromabwärts (3' nicht-codierende Sequenzen) einer codierenden Sequenz lokalisiert sein und die Transkription, das RNA-Processing oder die Stabilität und/oder die Translation der assoziierten codierenden Sequenz beeinflussen. Regulatorsequenzen können ohne Beschränkung Enhancer, Promotoren, Repressorbindungsstellen, Translationsleadersequenzen, Introns oder Polyadenylierungsignalsequenzen umfassen. Sie können natürliche und synthetische Sequenzen sowie Sequenzen umfassen, die eine Kombination aus synthetischen und natürlichen Sequenzen sind.

Der erfindungsgemäß verwendete Vektor kann auch geeignete Sequenzen zur Amplifikation der Expression umfassen.

Beispiele für Promotoren, die erfindungsgemäß verwendet werden können, sind Promotoren, von denen bekannt ist, dass sie die Expression in den eukaryotischen Zellen kontrollieren. Beliebige Promotoren mit der Fähigkeit zur Expression in Fadenpilzen können verwendet werden. Beispiele sind ein Promotor, der stark durch Stärke oder Zellulose induziert wird, z. B. ein Promotor für Glucoamylase oder $\alpha$-Amylase aus der Gattung *Aspergillus* oder Cellulase (Cellobiohydrolase) aus der Gattung *Trichoderma*, ein Promotor für Enzyme im glykolytischen Stoffwechselweg, wie beispielsweise Phosphoglyceratkinase (PGK) und Glycerinaldehyd-3-phosphat-dehydrogenase (GPD), etc. Bevorzugt ist der Cellobiohydrolase-I-, der Cellobiohydrolase-II-, der Amylase-, der Glucoamylase-, der Xylanase- oder der Enolase-Promotor.

Zusätzlich zur Verwendung eines speziellen Promotors können andere Typen von Elementen die Expression von Transgenen beeinflussen. Insbesondere wurde gezeigt, dass Introns das Potenzial zur Verstärkung der Transgenexpression besitzen.

Die Expressionskassette kann noch weitere Elemente umfassen, beispielsweise solche, die durch endogene oder exogene Elemente wie Zinkfinger-Proteine, einschließlich natürlich vorkommender Zinkfinger-Proteine oder chimärer Zinkfinger-Proteine, reguliert werden können.

Die erfindungsgemäß verwendete Expressionskassette kann ferner Enhancer-Elemente oder stromaufwärtige Promotor-Elemente enthalten.

Vektoren zur erfindungsgemäßen Verwendung können so konstruiert werden, dass sie ein Enhancer-Element enthalten. Die erfindungsgemäßen Konstrukte umfassen somit das Gen von Interesse zusammen mit einer 3'-DNA-Sequenz, die als Signal wirkt, um die Transkription zu terminieren und die Polyadenylierung der so erhaltenen mRNA zu erlauben. Es können beliebige Signalsequenzen verwendet werden, die die Sekretion aus dem gewählten Wirtsorganismus ermöglichen. Eine bevorzugte Signalsequenz ist die Phospholipase-Signalsequenz aus *Aspergillus fumigatus* oder daraus abgeleitete Signalsequenzen für die Sekretion aus filamentösen Pilzen.

Es kann auch eine spezielle Leadersequenz verwendet werden, da die DNA-Sequenz zwischen der Transkriptionsstartstelle und dem Start der codierenden Sequenz, d.h. der nicht-translatierten Leadersequenz die Genexpression beeinflussen kann. Bevorzugte Leadersequenzen umfassen Sequenzen, die die optimale Expression des angehefteten Gens steuern, d.h. sie umfassen eine bevorzugte Consensus-Leader-Sequenz, die die mRNA-Stabilität erhöht oder erhält und eine ungeeignete Translationsinitiation verhindert. Die Wahl solcher Sequenzen ist einem Fachmann auf dem Gebiet gut bekannt.

Um die Möglichkeit, der Identifikation der Transformanten zu verbessern, kann ein selektierbares oder screenbares Markergen in die Expressionskassette aufgenommen werden. Derartige Markergene sind einem Fachmann auf dem Gebiet gut bekannt.

Die Expressionskassette oder ein Vektorkonstrukt, das die Expressionskassette enthält, wird in eine Wirtszelle eingeführt. Eine Vielzahl von Techniken sind verfügbar und einem Fachmann auf dem Gebiet zur Einschleusung von Konstrukten in eine Wirtszelle gut bekannt. Die Transformation mikrobieller Zellen kann unter Verwendung von Polyethylenglykol, Calciumchlorid, viraler Infektion, DEAE-Dextran, Phageninfektionen, Elektroporation und anderen auf dem Fachgebiet bekannten Methoden durchgeführt werden. Die Transformation von Pilzen kann nach Penttilä et al., Gene 61:155-164, 1987 durchgeführt werden. Die Einschleusung eines rekombinanten Vektors in Hefen kann nach an sich bekannten Verfahren einschließlich Elektroporation, Verwendung von Sphäroplasten, Lithiumacetat und dergleichen durchgeführt werden.

Sobald die erfindungsgemäße Expressionskassette bzw. DNA-Sequenz erhalten ist, kann sie in Vektoren nach an sich bekannten Verfahren eingefügt werden, um das codierte Polypeptid in geeigneten Wirtssystemen zu überexprimieren. Jedoch können auch DNA-Sequenzen als solche verwendet werden, um geeignete Wirtssysteme der Erfindung zu transformieren, um eine Überexpression des codierten Polypeptids zu erzielen.

Sobald eine erfindungsgemäße DNA-Sequenz in einer geeigneten Wirtszelle in einem geeigneten Medium exprimiert wurde, kann die codierte Phospholipase entweder aus dem Medium, falls die Phospholipase in das Medium sezerniert wird oder aus dem Wirtsorganismus, falls die Phospholipase intrazellulär z. B. im periplasmatischen Raum vorhanden ist, nach an sich bekannten Verfahren aufkonzentriert und/oder isoliert werden. Bekannte Verfahren der Abtrennung der unlöslichen Bestandteile des Kulturmediums und der Biomasse gefolgt von Verfahren zur Aufkonzentrierung der Phospholipase können zur Herstellung von konzentrierten Phospholipaselösungen oder als Vorbereitung zur Trocknung der Phospholipase angewendet werden. Beispielsweise können Filtrationsverfahren oder Zentrifugationsverfahren zur Abtrennung der unlöslichen Bestandteile verwendet werden gefolgt von Ultrafiltrationsverfahren zur Aufkonzentration oder es werden Cross-flow-Filtrationsverfahren angewendet. Die Trocknung kann durch Gefrier- und Sprühtrocknen, Granulationsverfahren, Extrudierung oder andere Verfahren erfolgen. Bekannte Verfahren der Proteinreinigung können verwendet werden, um die erfindungsgemäßen Phospholipasen zu isolieren. Beispielsweise können verschiedene chromatographische oder gelchromatographische Verfahren einzeln oder in Kombination verwendet werden. In Abhängigkeit von der verwendeten Wirtszelle bei einem rekombinanten Produktionsverfahren kann das erfindungsgemäße Enzym kovalent durch Glykosilierung modifiziert sein oder nicht. In eukaryotischen Zellen dient die Glykosilierung der sezernierten Proteine dazu, die Proteinfaltung, die Konformationsstabilität, die thermische Stabilität und die Resistenz gegenüber Proteolyse zu modulieren. Im Hinblick auf eine spezifische Anwendung der Phospholipase kann eine glykosilierte Variante des Enzyms gegenüber einer nicht-glykosilierten Variante bevorzugt sein.

Die Erfindung betrifft auch isolierte oder im Wesentlichen gereinigte Nucleinsäure- oder Proteinzusammensetzungen. Dabei bezeichnet ein isoliertes und gereinigtes Polynucleotid/Polypeptid bzw. Segment davon ein Polynucleotid bzw. Polypeptid bzw. Segment davon, das isoliert aus seiner nativen Umgebung und in gereinigter Form zur weiteren Verwendung vorliegt. Ein isoliertes Polynucleinsäuresegment oder Polypeptid kann in gereinigter Form vorliegen oder kann in einer nicht nativen Umgebung vorliegen, wie beispielsweise in einer transgenen Wirtszelle. Beispielsweise ist ein isoliertes oder gereinigtes Polynucleotidsegment oder Protein oder ein biologisch aktiver Teil davon im Wesentlichen frei von weiterem zellulärem Material oder Kulturmedium bei Produktion nach rekombinanten Techniken oder im Wesentlichen frei von chemischen Vorläufern oder anderen chemischen Verbindungen. Bevorzugt ist ein isoliertes Polynucleotid frei von Sequenzen (bevorzugt Protein-codierenden Sequenzen) die natürlicherweise die Nucleinsäure (d.h. Sequenzen, die an den 5'- und 3'-Enden der Nucleinsäure lokalisiert sind) in der genomischen DNA des Organismus aus dem die Nucleinsäure stammt, flankieren. Beispielsweise kann gemäß verschiedenen Ausführungsformen das isolierte Nucleinsäuremolekül weniger als etwa 5 kb, 4 kb, 3 kb, 2 kb, 1 kb, 0,5 kb oder 0,1 kb Nucleotidsequenzen enthalten, die natürlicherweise das Nucleinsäuremolekül in der genomischen DNA der Zelle, aus der die Nucleinsäure abgeleitet ist, flankieren. Ein Protein, das im Wesentlichen frei von zellulärem Material ist, umfasst Zusammensetzungen von Protein oder Polypeptid mit weniger als etwa 70%, 50%, 30%, 20%, 10%, 5% (bezogen auf das Trockengewicht) von verunreinigendem Protein. Wenn das erfindungsgemäße Protein oder ein biologisch aktives Fragment davon rekombinant produziert wird, umfasst bevorzugt das Kulturmedium weniger als etwa 70%, 50%, 30%, 20%, 10% oder 5% (bezogen auf das Trockengewicht) der chemischen Vorläufer oder nicht-proteinartigen chemischen Substanzen.

Die Erfindung betrifft auch Phospholipasezusammensetzungen, die das erfindungsgemäße Polypeptid enthalten. Im Allgemeinen sind Phospholipasezusammensetzungen flüssig oder trocken. Flüssige Zusammensetzungen enthalten das Phospholipaseenzym bevorzugt in einer gereinigten oder angereicherten Form. Jedoch können auch Hilfsstoffe wie beispielsweise ein Stabilisator und/oder Glycerin, Sorbit oder Monopropylenglykol, Additive wie Salze, Zucker, Konservierungsstoffe, Mittel zur Einstellung des pH-Werts und Proteine zugesetzt werden. Typische Flüssigzusammensetzungen sind wässrige oder ölige Aufschlämmungen.

Trockenzusammensetzungen können gefriergetrocknete, sprühgetrocknete, granulierte oder extrudierte Zusammensetzungen sein, die ausschließlich das Enzym enthalten können. Trockenzusammensetzungen können Granulate sein, die leicht beispielsweise mit Nahrungsmitteln oder Futterkomponenten gemischt werden können oder bevorzugter eine Komponente eines Prämix bilden. Die Teilchengröße des Enzymgranulats ist bevorzugt mit der der anderen Komponenten des Gemisches kompatibel. Dies ermöglicht sichere und zweckdienliche Mittel, um Enzyme beispielsweise in prozessierte Nahrungsmittel, Prämixe oder Tierfutter einzuarbeiten.

Trockenzusammensetzungen können auch andere Additive wie z.B. Salze, insbesondere Phosphatsalze und deren Anhydroformen und Stabilisierungsmittel wie Polyvinylpyrrolidon etc. enthalten um bestimmte Bedingungen, wie z.B. den pH-Wert in der Anwendung zu regulieren.

Auf ähnliche Weise kann ein Nahrungsmitteladditiv gemäß dieser Ausführungsform der vorliegenden Erfindung mit anderen Nahrungsmittelkomponenten vereinigt werden, wodurch verarbeitete Nahrungsmittelprodukte erzeugt werden. Solche anderen Nahrungsmittelkomponenten umfassen ein oder mehrere Enzymsupplemente, Vitamine, Mineralien und Spurenelemente. Das so erhaltene kombinierte Nahrungsergänzungsmittel kann dann in einer geeigneten Menge mit anderen Nahrungsmittel-Komponenten wie Getreide und Pflanzenproteinen vermischt werden, um ein verarbeitetes Nahrungsmittel zu ergeben. Die Verarbeitung dieser Komponenten zu einem verarbeiteten Nahrungsmittel kann unter Verwendung an sich bekannter Verarbeitungsvorrichtungen durchgeführt werden.

In einer bevorzugten Ausführungsform umfassen die erfindungsgemäßen Phospholipasezusammensetzungen zusätzlich eine wirksame Menge eines oder mehrerer Enzyme für Nahrungs- oder Futtermittel oder für die Anwendung in Vorstufen zur Herstellung von Nahrungs- oder Futtermitteln, oder bei der Anwendung in der Textilindustrie, bevorzugt ausgewählt aus alpha-Galactosidasen, beta-Galactosidasen, Laccasen, anderen Phospholipasen, Phosphatasen, Endoglucanasen, insbesondere endo-beta-1,4-Glucanasen, endo-beta-1,3(4)-Glucanasen, endo-1,2-beta-Glucanasen und endo-1,3-alpha-Glucanasen, Cellulasen, Xylosidasen, Galactanasen, insbesondere Arabinogalactan-endo-1,4-beta-Galactosidasen und Arabinogalactan-endo-1,3-beta-Galactosidasen, Pectin-abbauenden Enzymen, insbesondere Pectinasen, Pectinesterasen, Pectinlyasen, Polygalacturonasen, Arabananasen, Rhamnogalacturonasen, Rhamnogalacturonanacetylesterasen, Rhamnogalacturonan-alpha-Rhamnosidasen, Pectatlyasen und alpha-Galacturonidasen, Mannanasen, beta-Mannosidasen, Mannanacetylesterasen, Xylanacetylesterasen, Proteasen, Xylanasen, Arabinoxylanasen, lipolytischen Enzymen wie Lipasen, Digalactosid-Diglycerid Esterasen und Cutinasen, und andere Enzyme wie Laccasen und Transglutaminasen.

Die erfindungsgemäßen Phospholipasen können für eine Vielzahl von Anwendungen verwendet werden. Beispiele hierfür sind Anwendungen beim Backen und in der Tierernährung sowie bei der Herstellung von Kraftstoffen aus erneuerbaren Energiequellen, zum Beispiel Rapssamen, bzw. in der Verarbeitung von Textilrohstoffen.

Eine bevorzugte Anwendung ist die Verwendung der erfindungsgemäßen Polypeptide mit Phospholipaseaktivität in Verfahren zur Entschleimung von pflanzlichem Öl. Dabei wird zum Beispiel das zu entschleimende Speiseöl mit einem erfindungsgemäßen Polypeptid behandelt, wodurch der Hauptteil der Phospholipide hydrolysiert wird, und anschließend wird die die hydrolysierten Phospholipide enthaltende wässrige Phase von dem Öl abgetrennt. Ein derartiges Verfahren eignet sich besonders zur Reinigung von Speiseölen, die Phospholipide enthalten, zum Beispiel von Pflanzenölen wie Sojabohnenöl, Rapssamenöl und Sonnenblumenöl.

Vor der Phospholipasebehandlung wird das Öl bevorzugt vorbehandelt, um Schleimstoffe zu entfernen, beispielsweise durch Feuchtraffinieren. Typischerweise enthält das Öl 50 bis 850 ppm Phosphor als Phospholipid zu Beginn der Behandlung mit der erfindungsgemäßen Phospholipase. Nach der Behandlung beträgt der Phosphorwert typischerweise zwischen 2 und 10 ppm.

Die Phospholipasebehandlung wird üblicherweise so durchgeführt, dass die Phospholipase in einer wässrigen Lösung dispergiert wird, bevorzugt als Tröpfchen mit einem durchschnittlichen Durchmesser von <10 μm. Die Menge an Wasser beträgt bevorzugt 0,5 bis 5 Gew.-% bezogen auf das Öl. Gegebenenfalls kann ein Emulgator zugesetzt werden. Es kann mechanisch gerührt werden, um eine Emulsion aufrechtzuerhalten. Die Behandlung mit Phospholipase kann bei einem pH-Wert im Bereich von etwa 3,5 bis etwa 5,0 durchgeführt werden. Der Verfahrens-pH-Wert kann im Bereich von etwa 3,5 bis etwa 5, bevorzugt 3,8 bis 4,5 und am bevorzugtesten 4,0 bis 4,2 liegen, um die Leistungsfähigkeit des Enzyms zu maximieren. Der pH-Wert kann beispielsweise durch Zugabe von Zitronensäure, einem Citratpuffer, Phosphorsäure oder Salzsäure eingestellt werden. Eine geeignete Temperatur beträgt allgemein 30°-70°C, bevorzug 45°-65°C und am bevorzugtesten 55°-62°C. Die Reaktionszeit beträgt typischerweise 1 bis 12 Stunden, bevorzugt 2 bis 6 Stunden. Eine geeignete Enzymdosierung beträgt üblicherweise 120 bis 3000 Einheiten pro kg Öl, bevorzugt 250 bis 2000 und am bevorzugtesten 750 bis 1500 Einheiten pro kg Öl.

Die Phospholipasebehandlung kann chargenweise, zum Beispiel in einem Tank unter Rühren, durchgeführt werden oder kann kontinuierlich sein, beispielsweise in einer Reihe von Tankreaktoren unter Rühren.

An die Phospholipasebehandlung schließt sich die Abtrennung einer wässrigen Phase und einer Ölphase an. Die Abtrennung kann durch herkömmliche Mittel, zum Beispiel Zentrifugation, durchgeführt werden. Die wässrige Phase enthält Phospholipasen und das Enzym kann zur Verbesserung der Ökonomie des Verfahrens erneut verwendet werden.

Die Behandlung kann unter Verwendung an sich bekannter Verfahren durchgeführt werden.

Die erfindungsgemäße Phospholipase kann auch vorteilhafterweise zur Herstellung von Teig und Backwaren verwendet werden, wobei in den Teig eine wirksame Menge eines erfindungsgemäßen Polypeptids eingearbeitet wird. Durch den Zusatz eines erfindungsgemäßen Polypeptids mit Phospholipaseaktivität können eine oder mehrere Eigenschaften des Teigs oder des aus dem Teig erhaltenen Backprodukts, verglichen mit einem Teig oder Backprodukt ohne Zusatz eines erfindungsgemäßen Polypeptids mit Phospholipaseaktivität, verbessert werden.

Bei der Teigbereitung unter Verwendung der erfindungsgemäßen Phospholipase kann die Phospholipase dem Teig selbst, jedem beliebigen Inhaltsstoff, aus dem der Teig hergestellt wird, und/oder einem Gemisch aus Teiginhaltsstoffen, aus denen der Teig hergestellt wird, zugesetzt werden. Ein erfindungsgemäßes Polypeptid mit Phospholipaseaktivität kann somit in jeder Stufe der Teigherstellung als solcher zugesetzt werden oder kann in ein, zwei oder mehreren Stufen zugesetzt werden. Eine wirksame Menge soll hier eine Menge an Phospholipase bezeichnen, die ausreicht einen messbaren Effekt auf mindestens eine Eigenschaft von Interesse des Teigs und/oder des Backprodukts hervorzurufen.

Der Ausdruck "verbesserte Eigenschaft" ist hier als jede Eigenschaft des Teigs und/oder eines Produkts, das aus dem Teig erhalten wird, insbesondere einer Backware, definiert, die durch die Wirkung der Phospholipase bezogen auf den Teig oder das Produkt, dem die erfindungsgemäße Phospholipase nicht zugesetzt wurde, verbessert wurde. Die verbesserte Eigenschaft kann beispielsweise umfassen: erhöhte Teigstärke, erhöhte Elastizität des Teigs, verbesserte Stabilität des Teigs, verringerte Klebrigkeit des Teigs, verbesserte Extensibilität des Teigs, verbesserte Maschinengängigkeit des Teigs, erhöhtes Volumen des Backprodukts, verbesserte Krumenstruktur des Backprodukts, verbesserte Weichheit des Backprodukts, verbessertes Aroma des Backprodukts und/oder verzögertes Altbackenwerdens des Backprodukts. Verfahren zur Bestimmung dieser Eigenschaften sind im Stand der Technik gut bekannt.

Ein Teig wird hier definiert als Gemisch aus Mehl und anderen Inhaltsstoffen, das fest genug ist um geknetet oder gewalzt zu werden. Der Teig kann frisch gefroren, vorgegart oder vorgebacken sein.

Der Ausdruck "Backprodukt" bezeichnet hier jedes Produkt, das aus einem Teig hergestellt wird und entweder einen weichen oder krossen Charakter besitzt. Beispiele für Backprodukte, die unter Verwendung einer erfindungsgemäßen Phospholipase hergestellt werden können, sind beispielsweise Brot (insbesondere Weißbrot, Vollkornbrot oder Roggenbrot), typischerweise in Form von Laiben oder Stangenbrot vom Typ französisches Baguette, Pasta, Pitabrot, Tortillas, Tacos, Kuchen, Pfannkuchen, Kekse und Kleingebäck, gekochtes Brot, doppelt gebackenes Brot und dergleichen.

Bei der Herstellung dieser Backwaren kann das erfindungsgemäße Polypeptid mit Phospholipaseaktivität und/oder ein oder mehrere weitere Enzyme in beliebigen Formulierungen zugesetzt werden, die für die jeweilige Verwendung geeignet sind, zum Beispiel in trockener Form, als Flüssigkeit oder als Prämix. Ferner können ein oder mehrere weitere Enzyme dem Teig ebenfalls zugesetzt werden. Diese weiteren Enzyme können beliebigen Ursprungs sein und zum Beispiel von Säugetieren und Pflanzen abstammen. Bevorzugt sind sie mikrobiellen Ursprungs und stammen besonders bevorzugt von Bakterien oder Pilzen ab.

Gemäß einer bevorzugten Ausführungsform können die weiteren Enzyme Amylasen wie α-Amylase (geeignet zur Erzeugung von Zuckern, die von Hefe fermentierbar sind, und zur Verzögerung des Altbackenwerdens) oder β-Amylase, Cyclodextringlucanotransferase, Peptidase, insbesondere eine Exopeptidase (geeignet zur Verstärkung des Aromas), Transglutaminase, Lipase (geeignet zur Modifikation der in dem Teig oder den Teigbestandteilen vorhandenen Lipide um den Teig weicher zu machen), Phospholipase (nützlich zur Modifikation der Lipide, die in dem Teig oder in den Teigbestandteilen vorhanden sind, um den Teig weicher zu machen und die Gasretention in dem Teig zu verbessern), Cellulase, Hemicellulase, insbesondere eine Pentosanase wie Xylanase (nützlich für die Teilhydrolyse von Pentosanen, die die Extensibilität des Teigs verbessern), Proteasen (nützlich für die Klebererweichung, insbesondere bei Verwendung von Hartweizenmehl), Proteindisulfidisomerase (beispielsweise eine Proteindisulfidisomerase, die in der WO 95/00636 offenbart ist), Glycosyltransferase, Peroxidase (nützlich zur Verbesserung der Konsistenz des Teigs), Laccase oder Oxidase, zum Beispiel eine Aldoseoxidase, Glucoseoxidase, Pyranooxidase, Lipoxygenase oder L-Aminosäureoxidase (nützlich zur Verbesserung der Konsistenz des Teigs) sein.

Diese(s) gegebenenfalls weiter zugesetzte(n) Enzym bzw. Enzyme kann bzw. können separat oder zusammen mit dem erfindungsgemäßen Polypeptid mit Phospholipaseaktivität gegebenenfalls als Bestandteile von Back- bzw. Teighilfsmittel zugesetzt werden. Die Erfindung betrifft auch die Herstellung solcher Teige sowie die Herstellung entsprechender Backwaren aus diesen Teigen.

Die Erfindung betrifft ferner auch einen Prämix, zum Beispiel in Form einer Mehlzusammensetzung, zur Herstellung von Teig und/oder Backwaren aus Teig, wobei dieser Prämix erfindungsgemäße Polypeptide mit Phospholipaseaktivität umfasst.

Die erfindungsgemäßen Polypeptide mit Phospholipaseaktivität können auch als Zusatz zu Tierfutter verwendet werden. Die Zugabe von Phospholipasen zu Futter verbessert die Effizienz der Futterverwertung bei Tieren. Dadurch wird das Wachstum der Tiere, die mit solchem Futter ernährt werden, verbessert. Dabei kann eine erfindungsgemäße Phospholipase als solche oder als Futterkonzentrat zugesetzt werden. Ferner kann die Phospholipase auch über transgene Pflanzen dem Tierfutter zugesetzt werden, worin die Phospholipase durch heterologe Genexpression synthetisiert wurde. Verfahren zur Herstellung derartiger transgener Pflanzen sind in der WO 91/14772 offenbart.

Die erfindungsgemäßen Polypeptide mit Phospholipaseaktivität können auch bei dem Prozess des Scouring in der Textilrohstoffverarbeitung von z.B. Baumwollfasern eingesetzt werden um die weitere Bearbeitung der Fasern zu erleichtern. Die durch das Scouring erzielten Verbesserungen wirken sich auf das Verhalten beim Anfärben wie auch die weitere mechanische und enzymatische Verarbeitung der Faser wie auch des daraus hergestellten Gewebes aus.

Das Gen für die aus dem Mikroorganismus *Aspergillus fumigatus* isolierte Phospholipase wurde in dem Plasmid B11B1Hind6 unter der Hinterlegungsnummer DSM 18369 am 14.06.2006 bei der Deutschen Sammlung von Mikroorganismen und Zellkulturen GmbH (DSMZ), Mascheroder Weg 1 b, D-38124 Braunschweig gemäß den Bedingungen des Budapester Vertrags hinterlegt.

Die Erfindung wird anhand der beigefügten Figuren näher erläutert. Es zeigen:

Figur 1: IEF Gel von gereinigter Phospholipase aus *Aspergillus fumigatus.* Die rechte Bahn (Nr. 6) enthält die Markerproteine aus dem Isoelectric Focusing Calibration Kit, pH 2,5-6,5, Fa. Pharmacia, No. 17-0472-01. Die Phospholipasebande auf der linken Bahn (Nr. 5) bei pl 4.4 ist mit dem Pfeil gekennzeichnet.

Figur 2: SDS-Gel der partiellen gereinigten Phospholipase aus *Aspergillus fumigatus* vor (Bahn 1 und 2) und nach Behandlung mit N-Glycosidase F (Bahn 4, 5, 6 und 7). Bahn 3 enthält die Markerproteins (SDS-PAGE Standards, low range: 97.400, 66.200, 45.000, 31.000, 21.500, 14.400, Biorad)

Figur 3: T-Optimumskurve für die Phospholipase aus *Aspergillus fumigatus* (RH 3949 IS15) Kulturüberstand und rekombinbant exprimiert in *Aspergillus niger* (RH 31019 und RH 31020).

Figur 4: pH-Optimumskurve für Phospholipase aus Kulturüberstand von *Aspergillus fumigatus* RH3949 IS15.

Figur 5: Nucleotidsequenz und daraus abgeleitete Aminosäuresequenz des chromosomalen Phospholipasegens aus *Aspergillus fumigatus* RH3949. Die Introns sind in Kursivschrift geschrieben. Die Übereinstimmung der Aminosäuresequenz mit den aus der Proteinsequenzierung ermittelten Peptidsequenzen ist unterstrichen (vgl. SEQ ID NOs: 1 und 2).

Figur 6: Die Nucleotidsequenz des chromosomalen Phospholipasegens aus *Aspergillus fumigatus* RH3949 (SEQ ID NO: 1).

Figur 7: Die Aminosäuresequenz des Phospholipasegens aus *Aspergillus fumigatus* RH3949 (SEQ ID NO: 2)

Figur 8: Restriktionskarte des Vektors B11B1Hind6

Figur 9: Restriktionskarte des Expressionsvektors pK3949/9

Figur 10: Restriktionskarte des Expressionsvektors pK3949/11

Die folgenden Beispiele erläutern die Erfindung näher:

**Referenzbeispiel 1**

**Bestimmung der Phospholipaseaktivität**

1 Phospholipase Unit entspricht der Enzymmenge, die unter Standardbedingungen 1 μmol Fettsäure pro Minute aus Phosphatidylcholin freisetzt.

**Reagenzien:**

**Substratiösung:**

1 g Epikuron 200 (gereinigtes Phosphatidylcholin aus Soja von LUCAS MEYER, BestNr. 139029), 100 ml deionisiertes-Wasser und 5 ml 0,32 M $CaCl_2$-Lösung werden mit einem *Ultra Turrax* 2 Min. bei 24000 UpM homogenisiert. Die Substratlösung ist bei 4°- 8°C 3 - 4 d haltbar.

**Andere Lösungen:**

0,32 M $MgCl_2$-Lösung, frische 3,3 mM Zitronensäure - Monohydrat- Lösung, 10 mM KOH-Lösung, 1%-ige Triton X100 (Fa. Fluka) Lösung in VE-Wasser

**Enzymlösung**

Die Enzympräparate werden in deionisiertem Wasser gelöst. Die Enzymkonzentration im Ansatz darf nicht über 2,5 U $g^{-1}$ enthalten.

**Durchführung der Bestimmung**

<u>Hauptwerte</u>

10 ml Substratlösung
10 ml 1%-ige Triton X100-Lösung
5 ml 3,3 mM Zitronensäure - Monohydrat- Lösung

werden in einen 25-ml-Weithalserlenmeyerkolben pipettiert und 10 min auf 40°C temperiert. Der pH-Wert stellt sich auf ca. 3,3 - 3,5 ein.

Nach der Zugabe von 0,1 ml Enzymlösung wird der Analysenansatz 10 min bei 40°C inkubiert. Nach Ablauf der Inkubationszeit wird mit 0,01 M KOH auf pH 10,0 titriert, wobei die ersten 5 ml KOH zügig (Dauer: ca.1 min) zugegeben werden. Der Verbrauch an KOH wird registriert.

<u>Blindwerte</u>

Die Enzymstammlösung wird 15 min bei 95°C erhitzt und somit deaktiviert. Nach dem Abkühlen auf Raumtemperatur erfolgt die weitere Behandlung wie bei den Hauptwerten.

Eine Inkubation der Blindwerte ist nicht notwendig.

<u>Auswertung:</u>

$$PLU / g = \frac{\Delta V_{KOH} * c_{KOH} * 1000}{\Delta t * c_s * v}$$

| | | |
|---|---|---|
| $v_{KOH}$ | [ml] | Verbrauchsdifferenz zwischen Blind- und Hauptwert |
| $c_{KOH}$ | [mol $l^{-1}$] | Konzentration der KOH |
| t | [min ] | Inkubationszeit |
| $c_s$ | [g $ml^{-1}$] | Konzentration der Probe |
| v | [ml] | eingesetztes Volumen |

**Beispiel 1**

**Herstellung von Phospholipase mit *Aspergillus fumigatus***

*Aspergillus fumigatus* RH3949 IS15 wurde in 200 ml Schüttelkolben, gefüllt mit 50 ml Medium bei 28°C, 200 UpM, über 5 d angezüchtet. Das Medium bestand aus 0,5% Epicuron 200 (Lucas Meyer), 0,5% Maisquellpulver, 0,2% $NH_4NO_3$, 100 mM $KH_2PO_4$ und 0,1% Triton X100. Der pH-Wert wurde vor dem Sterilisieren auf pH 6 eingestellt. Das Medium wurde mit einer Sporensuspension angeimpft. Nach 5 Tagen wurde der Kulturüberstand von dem Mycel durch Filtration getrennt und die Phospholipaseaktivität in der Flüssigkeit gemessen.

**Beispiel 2**

**Reinigung der Phospholipase von *Aspergillus fumigatus***

**Schritt 1: Anionenaustauscher**

Aufkonzentrierter Kulturüberstand aus den Züchtungen nach Beispiel 1 wurde über einen Anionenaustauscher in Proteinfraktionen getrennt.

Die Probe wurde 1,5 h im Dialyseschlauch (Naturin Eiweiß-Saitling) gegen vollentsalztes Wasser dialysiert. Der pH-Wert wurde mit 1 M NaOH auf pH 7 eingestellt. Die Phospholipase mit dem pI von ca. 4,4 wird nicht auf der Säule Macro Prep Q (Fa. BioRad 156-0051) adsorbiert wenn diese mit 20 mM Tris/HCl-Puffer pH 7 + 5 mM $CaCl_2$ äquilibriert ist, sondern wird im Durchlauf wiedergefunden.

**Schritt 2: HIC (hydrophobic interaction chromatography)**

Der Durchlauf des ersten Schrittes wurde mit konzentrierter Ammoniumsulfatlösung auf eine Ammoniumsulfatkonzentration von 1,7 M eingestellt. Die Probelösung wurde mit einer Ammoniumsulfatkonzentration von 1,7 M auf die HIC Säule, Phenyl Sepharose 6 Fast Flow low substitution (Fa. Pharmacia 17-0965-03), aufgetragen.

Die Phospholipase wurde im Durchlauf der mit 20 mM Tris/HCl-Puffer pH 7 + 5 mM $CaCl_2$ + 1,7 M Ammoniumsulfat äquilibrierten Säule gefunden.

**Schritt 3: Chromatofokussierung**

Der Durchlauf nach Schritt 2 wurde mit dem Jumbosep-Zentrifugenkonzentrator und 10 kDa Membraneinsatz (Fa. Pall Filtron, Nr. FD 010K65) mit dem Eluent A (0,025 M Histidin/HCl pH 6,2) umgepuffert und aufkonzentriert.

Die Probe wurde nun auf die Mono P, Mono P HR 5/20 (Fa. Pharmacia 17-0548-01), aufgetragen die für einen pH-Bereich von pH 6 - 4 äquilibriert ist [Eluent B Polypuffer 74 (Fa. Pharmacia 17-0713-01), pH 4].

Die Phospholipase wurde im Eluat wiedergefunden.

Die partiell gereinigte Phospholipase wurde auf ein IEF Gel aufgetragen (Figur 1). Zur Identifikation wurden die Banden ausgeschnitten und entsprechend der beschriebenen Analysenmethode auf Phospholipaseaktivität überprüft.

Die Phospholipasebanden wurden anschließend auch auf ein SDS-Gel aufgetragen, sowohl direkt wie auch nach Deglycosilierung mit N-Glycosidase F (New England BioLabs Inc.). Es wurden 2 Banden mit ca. 72 und 34 kDa bzw. nach Deglycosilierung mit ca. 54 und 20 kDa gefunden (Figur 2), die in Beispiel 4 weiter benutzt wurden.

**Beispiel 3**

**Charakterisierung der Phospholipasen aus *Aspergillus fumigatus***

Die Phospholipaseaktivität wurde mit der Bestimmungsmethode wie oben beschrieben bei verschiedenen Temperaturen ermittelt. Die Kurve in Figur 3 zeigt für das nativ mit Hilfe von *Aspergillus fumigatus* RH 3949 IS15 hergestellte Enzym (s. Beispiel 1) ein T-Optimum bei einer Temperatur von 45°C sowie für rekombinant mit Hilfe von *Aspergillus niger* Stämmen hergestelltes Enzym (s. Beispiel 5) ein T-Optimum von 50° - 52°C. Die Restaktivität bei 60°C wurde bei dem rekombinant hergestellten Enzym von 35% auf 87% erhöht.

Die Phospholipaseaktivität wurde mit der Bestimmungsmethode wie oben beschrieben bei verschiedenen pH-Werten ermittelt. Der pH-Wert wurde dazu mit Hilfe von Zitronensäure eingestellt. Die Kurve in Figur 4 zeigt ein pH-Optimum bei Werten von pH 3,5 und kleiner, sowie ein zweites lokales pH-Optimum bei pH 5 - 6 und damit für die Anwendung in der Ölentschleimung bei pH-Werten <5 optimale Eigenschaften um ein Ablagern von Ca-Verbindungen in der Zentrifuge zu vermeiden.

**Beispiel 4**

**Isolierung und Bestimmung der DNA-Sequenzen der Phospholipasen aus *Aspergillus fumigatus***

**a) N-Terminale Proteinsequenzierung**

Nach dem letzten Reinigungsschritt über die Mono P (Chromatofokussierung) wurden die Fraktionen mit höchster Phospholipaseaktivität gesammelt und auf einem Nativ-Gel getrennt. Die Proteinbande mit Phospholipaseaktivität wurde ausgeschnitten und erneut auf ein SDS-Gel aufgetragen. Dabei wurden zwei Fragmente, bezeichnet als B11 und B12 mit einem Molekulargewicht von ca. 72 bzw. 34 kDa gefunden. Nach Deglycosilierung mit N-Glycosidase F (New England BioLabs Inc.) zeigten diese Molekulargewichte von ca. 54 und 20 kDa. Die Proteinbanden, markiert in Figur 2 als B11 und B12, wurden auf eine PVDF- Membran (Fluotrans Transfer Membrane, Pall) transferiert und nach der Coomassie-Färbung die N-terminalen Aminosäuresequenzen in einem Aminosäure-Sequenzer (Applied Biosystems Model 470A) bestimmt. Sie lauten:

B11 : DSASY$^{5}$ YKDYS$^{10}$ NAVSG KAD$^{18}$ (SEQ ID NO: 3)

B12: ALPNA$^{5}$ PDGYT$^{10}$ PS-VG- PA$^{18}$ (SEQ ID NO: 4)

Beachtlich ist, dass die Bruchstücke B11 und B12 keine Übereinstimmung zu den Sequenzen bekannter Phospholipasen zeigen. Eine Ähnlichkeit liegt zu den Sequenzen von Lysophospholipasen vor. Um so überraschender war es, dass aufgrund der Bruchstücke B11 und B12 eine Phospholipase gefunden wurde.

**b) Bestimmung der Aminosäuresequenz von BrCN-Fragmente**

Die BrCN-Spaltung von Proteinen wurde nach einer Vorschrift von Gross (1967, The Cyanogen Bromide Reaction, Methods Enzymol, Vol. XI, 238-255) durchgeführt. Hierbei wurden die Proteinfragmente B11 und B12 nach der SDS-Gelelektrophorese aus dem Gel ausgeschnitten, mit 40% n-Propanol gewaschen und anschließend in einer Mischung von 750 mM BrCN in 70%-iger Ameisensäure für 24 h bei Raumtemperatur im Dunkeln inkubiert. Die Proteinfragmente im Überstand wurden nach der Entfernung des Bromcyans in der Vakuumzentrifuge in Puffer aufgenommen und danach zur Trennung kleiner Proteinfragmente auf ein SDS-Gel nach Schägger und Jagow (1987, Anal. Biochem. 199, 223-231) aufgetragen.

Nach der Gelelektrophorese wurden die Fragmente aus dem Gel auf eine PVDF-Membran transferriert (Polyvinylidene Difluoride Membranes), welche nach der Coomassie-Färbung identifiziert und zur Proteinsequenzierung eingesetzt wurden (Matsudaira, 1987, J. Biol. Chem. 262, 10035-10038).

Aus dem Protein B11 bzw. B12 wurden die Aminosäuresequenzen von folgenden BrCN-Fragmenten bestimmt:

B11/1 $^{1}$DSASY (SEQ ID NO: 5)
B11/2. $^{1}$PVVVA DGNYP$^{10}$ (SEQ ID NO: 6)
B11/5 $^{1}$-TSST LFNQF$^{10}$ (SEQ ID NO: 7)
B12/1 $^{1}$KDFFS HVKIQ$^{10}$ DFDAV GYID$^{19}$ (SEQ ID NO: 8)
B12/2 $^{1}$ALPNA (SEQ ID NO: 9)
B12/3 $^{1}$NTATA IKAFD$^{10}$ S-TP$^{14}$ (SEQ ID NO: 10)

**c) Synthese des 1-cDNA-Stranges**

Ca. 1 x $10^7$ Sporen des *A. fumigatus* Stammes RH 3949 IS15 wurden in 100 ml Medium (0,5% Maisquellpulver, 0,5% Epikuron 200, 0,1% Triton X100, 0,2% $NH_4NO_3$ und 100 mM $KH_2PO_4$ pH 6,0), beimpft und bei 45°C für 2 bis 3 Tage kultiviert. Das erhaltene Mycel wurde zur RNA-Präparation mittels der Qiagen-Säule (Qiagen) verwendet.

Die Synthese des 1-cDNA-Stranges erfolgte nach der Vorschrift des Herstellers (BRL). Hierbei wurden in einem 20 µl Reaktionsansatz 4 µl 5 x BRL Puffer (250 mM Tris/HCl, pH 8,3, 375 mM KCl, 15 mM $MgCl_2$), 1 µl 10 mM dNTP, 2 µl 100 mM DTT, 50 pMol Primer EA13, 1 µl RNA (2 µg gesamte RNA) und 2000 U RTase Super Script (BRL) zusammen pipettiert. Der Reaktionsansatz wurde für 50 min bei 45°C inkubiert.

Für die spätere Amplifikation der Phospholipase c-DNA mittels der Polymerase-Ketten-Reaktion wurde der Ansatz mit 20 µl bidest Wasser verdünnt und bei -0°C aufbewahrt.

Die DNA-Sequenz des Primers EA13 lautet:

5'-gAC TCg AgT CgA CAT CgA $(T)_{20}$ (A/C/g) -3' (SEQ ID NO: 11)

**d) Amplifizierung einer Phospholipase cDNA-Teilsequenz mittels der Polymerase Ketten Reaktion (PCR)**

Aus obigen Daten der Aminosäuresequenz wurden verschiedene Oligoprimer für die Amplifizierung der Phospholipase-cDNA abgeleitet. Die PCR-Produkte wurden in pGEMT-Plasmid kloniert und sequenziert. Dabei zeigt sich im Vergleich zu den Sequenzierdaten aus Beispiel 4 b), dass das Primerpaar B12/B5 und B12/B8 zu dem richtigen Phospholipase-cDNA Genfragment führt.

B12/B5 Primer
5'-gAC TTT gAC gCT gTg ggg TAC ATC gA-3" (SEQ ID NO: 12)

B12/B8 Primer
3'-TAC TTg TgA CgA Tgg CgT TAg TTC CgA AAA CT-5' (SEQ ID NO: 13)

Aus dem Ansatz der ersten cDNA Synthese wurde die Amplifizierung einer Phospholipase cDNA-Teilsequenz mittels der PCR Methode durchgeführt. Der Reaktionsansatz von 100 µl enthielt: 10 µl 10 x Puffer (200 mM Tris/HCl, pH 8,4, 500 mM KCl), 2 µl 10 mM dNTP, jeweils 50 pMol Oligoprimer (B12/B5 und B12/B8), 1 µl des 1.Strang-cDNA Ansatzes, 5U Taq DNA-Polymerase (BRL). Der Ansatz wurde für die Denaturierung bei 95°C für 5 min, 45 Zyklen (95°C für je 1 min, 45°C für 1 min, 72°C für 1 min) und anschließend die Extention bei 72°C für 5min durchgeführt.

Die PCR-Produkte wurden über Qiaquick-Säule gereinigt und in pGEMT-Plasmid kloniert.

Ein Transformant enthielt nach der Sequenzierung die richtige Phospholipase cDNA-Teilsequenz und wurde als B12/14/1 bezeichnet.

**e) Klonierung des chromosomalen Phospholipase Gens aus dem Stamm RH3949 IS15**

Die chromosomale DNA Präparation wurde nach einer Vorschrift von Hynes, M.J et al. (1983) Mol. Cell. Biol. 3, 1430-1439, durchgeführt.

Nach der Sau3A I partiellen Hydrolyse wurde die DNA durch eine Saccharose-Dichtegradientenzentrifugation nach ihrer Größe fraktioniert. Fraktionen, die DNA-Fragmente von 9-20 kb enthielten, wurden zusammengefaßt und mit Ethanol bei -0°C gefällt. Nach dem Waschen und Trocknen wurde die DNA in BamHI /EcoRI hydrolysierte EMBL3-DNA inseriert und in-vitro verpackt. Die Verpackung in das Phagenlysat Gigapack III Gold Packaging erfolgte nach der vom Hersteller beschriebenen Vorschrift (Stratagene Instruction Manual).

Zur Identifizierung des chromosomalen Phospholipase Gens in einer Lambda EMBL3-Genbank wurde das cDNA-Fragment aus dem Plasmid B12/14/1 als radioaktive Gensonde verwendet. Die Hybridisierung wurde bei 65°C, 18 h in Dextransulfat-Lösung (GenescreenPlus, DuPont) durchgeführt. Nach der Hybridisierung wurden die Filter jeweils 30 min zuerst mit 6 x SSC, zweimal 2x SSC, zweimal 2 x SSC, 0,1% SDS und anschließend mit 0, 2 x SSC bei 65°C (Membrane transfer and detection methods, Amersham) gewaschen.

Acht positive Klone wurden identifiziert. Aus den Ergebnissen der Analyse mit den Restriktionsendonucleasen und Southern-Hybridisierung mit der aus B12/14/1 isolierten Gensonde wurde die Phagen-DNA des Klons B1 mit HindIII hydrolysiert. Das in pUC18 inserierte 2,8 kb HindIII-Fragment wurde als B11B1Hind6 bezeichnet (Figur 8) und die Nucleotidsequenz des 2,8 kb DNA-Fragments wurde durch Sequenzierung bestimmt.

**f) Klonierung und Charakterisierung des N-terminalen Bereichs der Phosp holipase-cDNA**

Zur Ermittlung der Position des Initiationscodons bzw. der Signalsequenz des Phospholipase-Gens wurde die Amplifizierung des N-terminalen Bereichs der Phospholipase-cDNA mittels der PCR durchgeführt.

Die dafür verwendeten Oligoprimer B12A2P5 bzw. B12A2P9 wurden aus den Daten der chromosomalen DNA-Sequenz abgeleitet und synthetisiert.

B12A2P5 5'-CTT TGC GGC ACT GCG AAT-3' (SEQ ID NO: 14)
B12A2P9 5'-ATA TTT GAT CTT ATT GTC-3' (SEQ ID NO: 15)

Die PCR wurde unter den Bedingungen wie im Beispiel 4 e) durchgeführt. Das erhaltende PCR Produkt wurde in pGEMT-Vektor (Promega) kloniert und sequenziert.

Durch den Vergleich der cDNA- und chromosomalen Sequenz wurde das Vorhandensein und die Lage des Phospholipase ATG-Startcodons ermittelt. Die Bestimmung der Signalsequenz wurde nach einem Computerprogramm (PSORT) von Nakai und Kanehisa (1992, Genomics 14, 897-911) durchgeführt. Daraufhin weist das Phospholipase-Gen eine Signalsequenz von 20 Aminosäuren, ein mögliches Propeptid von 12 Aminosäuren und das Intron 1 mit 87 Basen auf.

**g) Klonierung des C-terminalen Bereichs der Phospholipase cDNA**

Im gleichen Verfahren wie unter dem Beispiel 4 f) wurde der C-terminale Bereich der Phospholipase cDNA amplifziert. Die dazu verwendeten Oligoprimer haben folgende Sequenz:

B11B1P15 5'-GGC GCA GGT GCG ATT AAG GCT TTT GA -3' (SEQ ID NO: 16)
B11B1P13 5'-TTC GCG AAA CTC TCG AAA TGA ATT CTA-3' (SEQ ID NO: 17)

Das erhaltene PCR-Produkt wurde in dem pGEMT-Vektor kloniert, sequenziert und als cDNA 4/20 bezeichnet. Durch den Vergleich der Phospholipase cDNA- und chromosomaler DNA-Sequenz wurde die Lage des Introns 2 mit 56 Basen im Phospholipase-Gen bestätigt.

**h) Konstruktion des Expressionsvektors pK3949/9**

In dem Expressionsvektor pK3949/9 (Figur 9) steht das Phospholipase Gen ohne Intron 2 unter der Kontrolle des *A. oryzae* α-Amylase Promotors.

Die Konstruktion des Expressionsvektors pK3949/9 erfolgt in drei Schritten:

- Einführen einer BspHI-Schnittstelle an dem Startcodon mittels der PCR Methode.

Die dafür verwendeten Primer haben folgende Sequenz:

Primer B11B1N3
5'-CGC GGA TCC GTC ATG AAG TCC ATC GCA GTG GCG TGC -3' (SEQ ID NO: 18)

Primer B11/B11
5'-TTG ACT AGT TTG AAC CAC ACT TCA AG-3' (SEQ ID NO: 19)

Die PCR erfolgt unter den Bedingungen wie im Beispiel 4 d). Das PCR-Produkt wurde mit den Enzymen BamHI/SpeI hydrolysiert und anschließend in das mit den selben Enzymen hydrolysierte B11B1Hind6 inseriert. Das erhaltene Plasmid hat die Bezeichnung pK3949/1.

- Aus dem Plasmid pK3949/1 wurde das Phospholipase-Gen als BspHI/HindIII Fragment in das mit NcoI/HindIII geschnittene Plasmid pK54 eingebaut. Das erhaltene Plasmid pK3949/2 enthält das Phospholipase Gen unter der Kontrolle des A. oryzae α-Amylase Promotors.

Das Plasmid pK54 enthält die Promotorsequenz des *A. oryzae* α-Amylasegens. Die α-Amylase Promotorsequenz wurde aus *A. oryzae* DSM63303 isoliert (Wirsel et al. 1989, Mol. Microbiol. 3 (1), 3-14), mittels PCR modifiziert und enthält eine NcoI-Schnittsstelle unmittelbar upstream des ATG-Codons.

- Durch den Austausch des PpuMI/StyI Fragments gegen das aus dem Plasmid cDNA 4/20 isolierten PpuMI/StyI Fragment wurde der Expressionsvektor pK3949/9 konstruiert (Figur 9).

**i) Konstruktion des Expressionsvektor pK3949/11**

In dem Vektor pK3949/11 (Figur 10) wurde das Intron 1 und das Propeptid deletiert, sodass das Phospholipase Gen mit eigener Signalsequenz direkt an dem *A. oryzae* α-Amylase Promotor fusioniert. Als Ausgangsplasmid wurde der Vektor pK3949/2 verwendet. Die Isolierung einzelner Fragmente wurde mittels der PCR-Methode durchgeführt, wobei die Reaktionsbedingungen wie unter dem aufgeführten Beispiel 6 d) eingehalten wurden. Folgende Primer wurden verwendet:

K17 5'-GAA TTC TGG TGT TTT GAT CTT TT-3' (SEQ ID NO: 20)
K18 5'-AGC ACC GCT AGC ACC GGA CAA TAA TAG GCC GGC GAC-3' (SEQ ID NO: 21)

K19 5'-TCC GGT GCT AGC_GGT GCT GCC CTG CCC AAT GCC CCC GAT GGA TAC ACA-3' (SEQ ID NO: 22)
K20 5'-GAA GTC CTT CAT CGC AGA AGT-3' (SEQ ID NO: 23)

K21 5'-CTG ATA TTT ACG TAA AAA TCG TCA-3' (SEQ ID NO: 24)
K22 5'-CTT GCC TCG ACG CGT CTG AAG CCA TGA-3' (SEQ ID NO: 25)

Mit den Primerpaaren K17/K18 wurden die Genabschnitte bestehend aus *A. ory*zae α-Amylase Promotor und Phospholipase Signalsequenz amplifiziert. Mit den Primerpaaren K19/K20 wurde die Genabschnitte bestehend aus der Phospholipase Signalsequenz und dem N-terminalen Teil des Phospholipase-Gens amplifiziert. Durch Austausch der Basen wurde die NheI-Schnittstelle eingeführt ohne Änderung der Aminosäurezusammensetzung. Die PCR-Produkte wurden gereinigt, mit NheI hydrolysiert und zusammenligiert. Das Ligationsprodukt dient als Matrix und die Oligos K21 und K22 als Primer für den zweiten PCR-Ansatz. Das erhaltene PCR-Fragment wurde nach der Reinigung mit SnaBI/MluI hydrolysiert und anschließend in das mit den gleichen Enzymen geschnittene Plasmid pK3949/2 inseriert. Der erhaltene Vektor erhält die Bezeichnung pK3949/11 (Figur 10).

**Beispiel 5**

**Transformation von *A. niger* NRRL3 mit DNA aus *Aspergillus fumigatus***

Die Isolierung von Protoplasten und die Transformation von *A. niger* wurde nach der Methode von Yelton et al. 1984, Proc. Natl. Acad. Sci. USA 81, 1470-1474 durchgeführt.

Als Selektionsplasmid wurde das Plasmid pAN7-1 (Punt et al., 1987, Gene 56, 117-124) für die Co-Transformation von *A. niger* verwendet.

10 μg Selektionsplasmid und 10 μg Expressionsplasmid wurden zusammen in 20 μl $H_2O$ in einem Eppendorfgefäß vorgelegt. Zu der Plasmidlösung wurden 200 μl Protoplastensuspension (ca $2 \times 10^7$ Protoplasten) gegeben, durch Invertieren vorsichtig gemischt und anschließend 5 min bei RT inkubiert. Nach Zugabe von 50 μl PTC-Lösung (60% Polyethylenglykol 6000, 10 mM Tris/HCl pH 7,5, 50 mM $CaCl_2$) erfolgte eine 20 min Inkubation bei RT. Nach nochmaliger Zugabe von 750 μl PTC-Lösung und einer weiteren 20 min Inkubation bei RT wurde der Ansatz 1 bis 2 min in der Eppendorfzentrifuge zentrifugiert. Die Protoplasten wurden in 1 ml STC-Lösung (1,0 M Sorbitol, 10 mM Tris/HCl pH 7,5, 50 mM $CaCl_2$) sorgfältig resuspendiert und auf 10 - 15 Selektionsagarplatten ausplattiert (per Liter: 33,4 g Czapek-Dox-Liquid-Medium (Oxoid), 1 M Saccharose und 12 g hochreiner Agar No 1 (Oxoid) und 100 mg Hygromycin B (Sigma).

Die Platten wurden danach bei 30°C 5 bis 7 d bis zur Sporulation inkubiert. Um genetisch reine Klone zu erhalten wurden die Transformanten noch zweimal auf Selektionsagarplatten vereinzelt. Drei Transformanten RH 31019, RH 31021 und RH 31025 wurden für weitere Experimente ausgewählt.

**Beispiel 6 (Referenzbeispiel)**

**Entschleimung von Öl**

In Rapsöl mit 535 ppm Phosphorgehalt wurde in einem ersten Schritt durch Wasserentschleimung der Phospholipidgehalt auf 120 ppm Phosphor reduziert um anschließend in einem zweiten Schritt durch Enzymzugabe den Phospholipidgehalt weiter zu senken. Hierbei wurde die Wasserphase der Wasserentschleimung nicht verworfen, sondern verblieb im Reaktionsansatz. Eine Abtrennung ist auch möglich.

250 g Rapsöl wurden in einen Dreihalsrundkolben eingefüllt und durch Einschalten der Kreiselpumpe (Metabo Vorsatzpumpe 27621) im Kreis geführt bis das Öl die Reaktionstemperatur von 60°C erreicht hatte. Dann erfolgte die Zugabe der Zitronensäure zum Öl auf eine Endkonzentration von 0.1% (w/v). Nach 120 min wurde der pH-Wert durch Zugabe von 7%-iger NaOH Lösung auf pH 4.0 eingestellt um optimale Arbeitsbedingungen für das Enzym bereitzustellen. Nach Erreichen der Reaktionstemperatur von 60°C bis 65°C wurde die Enzymlösung zugegeben, so dass Enzymaktivitäten von 250 bis 3000 PLU je kg Rohöl resultierten. Der Gesamtwassergehalt aller wässrigen Dosagen (Zitronensäure, NaOH, Enzymlösung) beträgt 1 % bis 5% im Öl. Zwischen den Zugaben wurde der Reaktionsansatz gut gemischt. Der Kolben wurde immer fest verschlossen um eine Verdunstung des Wassers zu vermeiden. Die Probenentnahme von 20 ml erfolgte alle 120 min nach Zugabe der Enzymlösung. Die Proben wurden 5 min bei 4300 x g zentrifugiert und der Phospholipidgehalt wurde, ausgedrückt in ppm Phosphor, im Öl nach Veraschung bei 850°C unter Zugabe von Magnesiumoxid, als Phosphomolybdatkomplex photometrisch bei 830 nm bestimmt.

Der Phospholipidgehalt kann auch flammenphotometrisch mit Hilfe eines AAS-Gerätes direkt im Öl bestimmt werden.

**Beispiel 7**

**Ergebnisse mit Enzym aus Kulturüberständen von *A. fumigatus* RH 3949 IS15**

Kulturüberstände von RH 3949 IS15 aus Beispiel 1 wurden bei verschiedenen Temperaturen (62,5° - 65°C) benutzt um Rapsöl nach Beispiel 6 zu entschleimen. Alle Versuche liefen bei pH 4 und einem Gesamtwassergehalt von 5%.

**Tab.: 1: Entschleimung von Rapsöl (605 ppm P) mit Phospholipase-Enzym aus Kulturüberständen von *Aspergillus fumigatus* RH3949 IS15 und** 5% **Wassergehalt bei pH 4,0.**

| Probenbezeichnung | Zeit [min] | 62,5°C [ppm P] | 64°C [ppm P] | 65°C [PPm P] |
|---|---|---|---|---|
| **Zitronensäure** | 90 | 115,4 | 115,4 | 115,5 |
| | 180 | 106,0 | 106,0 | 101,1 |
| | 270 | 85,7 | 85,7 | 87,0 |
| | 360 | 79,2 | 79,2 | 73,7 |
| **500 PLU $kg^{-1}$ Rohöl** | 90 | 76,1 | 80,0 | 97,7 |
| | 180 | 18,0 | 51,3 | 80,4 |
| | 270 | 12,6 | 35,1 | 64,6 |
| | 360 | 10,4 | 30,0 | 53,7 |
| **1000 PLU $kg^{-1}$ Rohöl** | 90 | 35,2 | 27,3 | 60,7 |
| | 180 | 13,3 | 10,8 | 31,6 |
| | 270 | 10,0 | 8,8 | 23,3 |
| | 360 | 7,2 | 9,5 | 20,0 |

Die Ergebnisse zeigen eine deutliche Entschleimungswirkung durch das Enzym im Vergleich zur Wasserentschleimung mit Zitronensäure. Die Wirkung ist auch von der Dosage abhängig (Vergleich 500 PLU $kg^{-1}$ zu 1000 PLU $kg^{-1}$) und das Enzym kann bis zu Temperaturen von 65°C eingesetzt werden. Die Hitzestabilität des Enzyms ist in der Ölentschleimung damit deutlich höher als bei der Bestimmung in wässriger Lösung wie in Beispiel 3 vorgenommen.

Durch die in Beispiel 6 beschriebene Abtrennung der Wasser- und Schlammphase kann die enzymhaltige Fraktion wiedergewonnen werden und erneut einem Versuch zur Ölentschleimung zugesetzt werden. Die nachfolgende Tabelle zeigt die Ergebnisse von bis zu fünf Wiederholungen.

**Tab.: 2: Entschleimung von Rapsöl (605 ppm P) mit Phospholipase-Enzym aus Kulturüberständen von *Aspergillus fumigatus* RH3949 IS15 und 5% Wassergehalt bei pH 4,0, 60°C und 1000 PLU je kg Rohöl, die dem ersten Zyklus zugesetzt wurden. In jedem weiteren Zyklus wurde nur die wässrige Phase (Wasser und Schlamm) nach Zentrifugation des vorangegangenen Zyklus eingesetzt.**

| Zyklus | [ppm P] nach 6 h |
|---|---|
| 1 | 5,4 |
| 2 | 6,0 |

(fortgesetzt)

| Zyklus | [ppm P] nach 6 h |
|---|---|
| 3 | 6,3 |
| 4 | 13,6 |
| 5 | 27,4 |

Die Ergebnisse zeigen, dass das Enzym mehr als 3 mal (d.h. >18 h) eingesetzt werden kann ohne dass eine signifikante Inaktivierung auftritt.

**Beispiel 8**

**Ergebnisse mit Enzym aus Kulturüberständen von rekombinanten *Aspergillus niger* Stämmen mit dem Gen aus *A. fumigatus* RH 3949 IS15**

Die rekombinanten A. *niger* NRRL3 Stämme aus Beispiel 5, die die Plasmide B11B1Hind6, pK3949/9 und pK3949/11 enthalten, haben die Bezeichnungen RH31019, RH31021 und RH31025. In Tabelle 3 sind die Entschleimungsergebnisse von Rapsöl mit diesen Stämmen aufgelistet. Dabei zeigt sich, dass auch das rekombinant mit *Aspergillus niger* RH31025 hergestellte Enzym die Hitzestabilität des mit dem wild-typ Stamm *Aspergillus fumigatus* RH3949 IS15 hergestellten Enzyms hat.

**Tab.: 3: Entschleimung von Rapsöl (535 ppm P) mit Phospholipase-Enzym (1000 PLU je kg Rohöl) aus Kulturüberständen von rekombinanten *A. niger* Stämmen mit 5% Wassergehalt bei pH 4,0. (n.d. nicht bestimmt)**

| **Probenbezeichnung** | **Zeit** [min] | **60°C** [ppm P] | **64°C** [ppm P] |
|---|---|---|---|
| **Zitronensäure** | 90<br>180<br>270<br>360 | 84,6<br>72,7<br>53,0<br>49,7 | 67,9<br>47,2<br>41,3<br>38,5 |
| **RH31019** | 90<br>180<br>270<br>360 | 53,2<br>30,7<br>18,5<br>8,8 | n.d.<br>n.d.<br>n.d.<br>n.d. |
| **RH31021** | 90<br>180<br>270<br>360 | 63,3<br>39,6<br>34,5<br>16,0 | n.d.<br>n.d.<br>n.d.<br>n.d. |
| **RH31025** | 90<br>180<br>270<br>360 | 49,3<br>25,7<br>16,7<br>11,9 | 26,8<br>13,9<br>6,8<br>4,8 |

## Patentansprüche

**1.** DNA-Sequenz, die ein Polypeptid mit Phospholipaseaktivität codiert, **dadurch gekennzeichnet, dass** die DNA-Sequenz ausgewählt ist aus

a) DNA-Sequenzen, die eine Nucleotidsequenz gemäß SEQ ID NO: 1 umfassen,
b) DNA-Sequenzen, die die codierende Sequenz gemäß SEQ ID NO: 1 umfassen,
c) DNA-Sequenzen, die die Aminosäuresequenz gemäß SEQ ID NO: 2 codieren,
d) DNA-Sequenzen, die von dem Plasmid B11 B1 Hind6 mit der Restriktionskarte gemäß Figur 8 und hinterlegt unter der Hinterlegungsnummer DSM 18369 codiert werden, und
e) komplementären Strängen zu den Sequenzen gemäß a) bis d),

wobei die DNA-Sequenz bevorzugt aus *Aspergillus* und noch bevorzugter aus *Aspergillus fumigatus* abgeleitet ist.

**2.** Nucleinsäuresequenz, umfassend ein Analogon einer der Sequenzen gemäß Anspruch 1, **dadurch gekennzeichnet, dass** die Sequenz ein Polypeptid mit Phospholipaseaktivität codiert und

a) *mindestens* 92% Identität zu einer dieser Sequenzen aufweist, oder
b) ein komplementärer Strang zu den Sequenzen gemäß a) ist,

wobei die Sequenz erhältlich ist durch Substitution, Deletion, Insertion, Addition oder Mutation einer oder mehrerer Nucleinsäuren der DNA-Sequenz gemäß SEQ ID NO: 1.

**3.** Expressionskonstrukt umfassend eine Sequenz nach einem der Ansprüche 1 bis 2 in funktioneller Verknüpfung mit einer oder mehreren Sequenz(en) zur Steuerung der Expression des Polypeptids mit Phospholipaseaktivität in einer geeigneten Wirtszelle, wobei die Sequenz zur Steuerung der Expression des Polypeptids bevorzugt ein Promotor, ausgewählt aus dem Glucoamylase- oder $\alpha$-Amylase-Promotor der Gattung *Aspergillus,* dem Cellulase (Cellobiohydrolase-)-Promotor der Gattung *Trichoderma,* einem Promotor für ein Enzym im glykolytischen Stoffwechselweg wie Phosphoglyceratkinase oder Glycerinaldehyd-3-phosphatdehydrogenase, dem Xylanasepromotor oder dem Enolase-Promotor ist, und gegebenenfalls umfassend weiterhin eine sekretorische Leadersequenz.

**4.** Rekombinante Wirtszelle, **dadurch gekennzeichnet, dass** sie ein Expressionskonstrukt nach Anspruch 3 enthält.

**5.** Rekombinante Wirtszelle nach Anspruch 4, **dadurch gekennzeichnet, dass** sie von einer Pilzzelle der Gattung *Aspergillus Rhizopus, Trichoderma, Neurospora, Mucor* oder *Penicillium* oder einer Hefezelle der Gattung *Kluyveromyces, Saccharomyces, Schizosaccharomyces, Trichosporon, Schwanniomyces, Hansenula* oder *Pichia* abgeleitet ist.

**6.** Plasmid B11B1Hind6 mit der Restriktionskarte gemäß Figur 8 und hinterlegt unter der Hinterlegungsnummer DSM 18369.

**7.** Polypeptid mit Phospholipaseaktivität ausgewählt aus

a) einem Polypeptid, das von dem codierenden Teil einer DNA-Sequenz nach einem der Ansprüche 1 bis 2 codiert wird,
b) einem Polypeptid mit der Sequenz gemäß SEQ ID NO: 2,
c) einem Polypeptid mit einer Sequenz, die mindestens 92% Identität zu den Aminosäuren 33 bis 633 von SEQ ID NO: 2 aufweist.

**8.** Phospholipasezusammensetzung, **dadurch gekennzeichnet, dass** sie ein Polypeptid nach Anspruch 7 zusammen mit Hilfsstoffen umfasst und dass sie gegebenenfalls weiterhin ein oder mehrere Enzyme für Nahrungs- oder Futtermittel umfasst.

**9.** Verwendung eines Polypeptids nach Anspruch 7 oder einer Phospholipasezusammensetzung nach Anspruch 8 zur Entschleimung von pflanzlichem Öl, zur Herstellung von Teig und/oder Backwaren, zur Herstellung von Milchprodukten, als Zusatz zu Tierfutter oder zur Bearbeitung von Textilrohstoffen.

**10.** Verfahren zur Produktion eines Polypeptids mit Phospholipaseaktivität nach Anspruch 7, **dadurch gekennzeichnet, dass** man eine Wirtszelle nach einem der Ansprüche 4 oder 5 unter Bedingungen, die die Expression des Polypeptids fördern, züchtet und anschließend das Polypeptid gewinnt.

**11.** Verfahren zur Entschleimung von pflanzlichem Öl, **dadurch gekennzeichnet, dass** man

a) das zu behandelnde pflanzliche Öl gegebenenfalls vorbehandelt,
b) dem so gegebenenfalls vorbehandelten Öl eine wässrige ein Polypeptid nach Anspruch 7 enthaltende Lösung oder eine Phospholipasezusammensetzung nach Anspruch 8 zusetzt,
c) den Reaktionsansatz nach b) 1 bis 12 h auf eine Temperatur zwischen 30° und 70°C erhitzt, und
d) die wässrige und ölige Phase voneinander trennt.

## Claims

**1.** A DNA sequence that encodes a polypeptide with phospholipase activity, **characterised in that** the DNA sequence is selected from

a) DNA sequences that comprise a nucleotide sequence according to SEQ ID NO:1,
b) DNA sequences that comprise the encoding sequence according to SEQ ID NO:1,
c) DNA sequences that encode the amino acid sequence according to SEQ ID NO:2,
d) DNA sequences encoded by the plasmid B11B1Hind6 having the restriction map according to Fig. 8 and deposited under accession no. DSM 18369, and
e) strands complementary to the sequences according to a) to d)

the DNA sequence preferably being derived from *Aspergillus* and even more preferably from *Aspergillus fumigatus.*

**2.** A nucleic acid sequence comprising an analogue of one of the sequences according to claim 1, **characterised in that** the sequence encodes a polypeptide with phospholipase activity and

a) has *at least* 92% identity to one of these sequences, or
b) is a strand complementary to the sequences according to a),

said sequence being obtainable by substitution, deletion, insertion, addition or mutation of one or more nucleic acids of the DNA sequence according to SEQ ID NO: 1.

**3.** An expression construct comprising a sequence according to one of the claims 1 to 2 in functional linkage with one or more sequence(s) to control the expression of the polypeptide with phospholipase activity in an appropriate host cell, the sequence for controlling the expression of the polypeptide preferably being a promoter selected from the glucoamylase or $\alpha$-amalyse promoter of the genus *Aspergillus,* the cellulase (cellobiohydrolase) promoter of the genus *Trichoderma,* a promoter for an enzyme in the glycolytic metabolic path such as phosphoglycerate kinase or glycerol aldehyde-3-phosphate dehydrogenase, the xylanase promoter or the enolase promoter, optionally also comprising a secretory leader sequence.

**4.** A recombinant host cell, **characterised in that** it contains an expression construct according to claim 3.

**5.** A recombinant host cell according to claim 4, **characterised in that** it is derived from a fungus cell of the genus *Aspergillus rhizopus, Trichoderma, Neuospora, Mucor,* or *Penicillium* or a yeast cell of the genus *Kluyveromyces, Saccharomyces, Schizosaccharomyces, Trichosporon, Schwanniomyces, Hansenula* or *Pichia.*

**6.** The plasmid B11B1Hind6 having the restriction map according to Fig. 8 and deposited under accession no. DSM 18369.

**7.** A polypeptide with phospholipase activity, selected from

a) a polypeptide encoded by the encoding part of a DNA sequence according to any of the claims 1 to 2,
b) a polypeptide having the sequence according to SEQ ID NO:2,
c) a polypeptide having a sequence that has at least 92% identity to amino acids 33 to 633 of SEQ ID NO: 2.

**8.** A phospholipidase composition, **characterised in that** it comprises a polypeptide according to claim 7 together with adjuvants and optionally further comprises one or more enzymes for food or feed products.

**9.** The use of a polypeptide according to claim 7 or a phospholipidase composition according to claim 8 for degumming of vegetable oil, for the preparation of dough and/or bakery products, the preparation of dairy products, as an additive to animal feed or for processing textile raw materials.

**10.** A method for producing a polypeptide with phospholipase activity according to claim 7, **characterised in that** a host cell according to any of the claims 4 or 5 is cultivated under conditions that support the expression of the polypeptide, and subsequently the polypeptide is recovered.

**11.** A method for degumming of vegetable oil, **characterised in that**

a) the vegetable oil to be treated is optionally pre-treated,
b) an aqueous solution containing a polypeptide according to claim 7 or a phospholipase composition according to claim 8 is added to the optionally pre-treated oil,
c) the reaction batch according to b) is heated to a temperature between 30 and 70°C for 1 to 12 hours, and
d) the aqueous phase is separated from the oily phase.

## Revendications

**1.** Séquence d'ADN qui code un polypeptide ayant une activité de phospholipase, **caractérisée en ce que** la séquence d'ADN est choisie parmi :

a) les séquences d'ADN qui comprennent une séquence nucléotidique selon la SEQ ID N°1,
b) les séquences d'ADN qui comprennent la séquence codante selon la SEQ ID N°1,
c) les séquences d'ADN qui codent la séquence d'acides aminés selon la SEQ ID N°2,
d) les séquences d'ADN qui sont codées par le plasmide B11B1Hind6 ayant la carte de restriction selon la figure 8 et déposé sous le numéro d'enregistrement DSM 18369, et
e) des brins complémentaires aux séquences selon a) à d),

dans laquelle la séquence d'ADN est de préférence dérivée de *Aspergillus* et encore plus préférentiellement de *Aspergillus fumigatus.*

**2.** Séquence d'acide nucléique, comprenant un analogue d'une des séquences selon la revendication 1, **caractérisée en ce que** la séquence code un polypeptide ayant une activité de phospholipase et

a) présente une identité d'au moins 92 % à l'une de ces séquences ou
b) est un brin complémentaire aux séquences selon a),

la séquence pouvant être obtenue par substitution, délétion, insertion, addition ou mutation d'un ou de plusieurs acides nucléiques de la séquence d'ADN selon la SEQ ID N°1.

**3.** Hybride d'expression comprenant une séquence selon l'une des revendications 1 à 2 en relation fonctionnelle avec une ou plusieurs séquences pour le contrôle de l'expression du polypeptide ayant une activité de phospholipase dans une cellule hôte appropriée, la séquence pour le contrôle de l'expression du polypeptide étant de préférence un promoteur, choisi parmi le promoteur de la glucoamylase ou $\alpha$-amylase du genre *Aspergillus,* le promoteur de la cellulase (cellobiohydrolase) du genre *Trichoderma,* un promoteur pour une enzyme dans la voie métabolique glycolytique comme la phosphoglycérate kinase ou la glycérinaldéhyde-3-phosphate hydrogénase, le promoteur de la xylanase ou le promoteur de l'énolase, et comprenant le cas échéant en outre une séquence leader sécrétoire.

**4.** Cellule hôte recombinante, **caractérisée en ce qu'**elle comprend un hybride d'expression selon la revendication 3.

**5.** Cellule hôte recombinante selon la revendication 4, **caractérisée en ce qu'**elle est dérivée d'une cellule fongique du genre *Aspergillus Rhizopus, Trichoderma, Neurospora, Mucor* ou *Penicillium* ou d'une cellule de levure du genre *Kluyveromyces, Saccharomyces, Schizosaccharomyces, Trichosporon, Schwanniomyces, Hansenula* ou *Pichia.*

**6.** Plasmide B11B1Hind6 ayant la carte de restriction selon la figure 8 et déposé sous le numéro d'enregistrement DSM 18369.

**7.** Polypeptide ayant une activité de phospholipase choisi parmi

a) un polypeptide, qui est codé par la partie codante d'une séquence d'ADN selon l'une des revendications 1 à 2,
b) un polypeptide ayant une séquence selon la SEQ ID N°2,
c) un polypeptide ayant une séquence qui présente une identité d'au moins 92 % avec les acides aminés 33 à 633 de la SEQ ID N°2.

**8.** Composition de phospholipase, **caractérisée en ce qu'**elle comprend un polypeptide selon la revendication 7 conjointement avec des auxiliaires et qu'elle comprend le cas échéant en outre une ou plusieurs enzymes pour des produits alimentaires ou des aliments pour animaux.

**9.** Utilisation d'un polypeptide selon la revendication 7 ou d'une composition de phospholipase selon la revendication 8 pour la démucilagination de l'huile végétale, pour la fabrication de pâte et/ou de gâteaux, pour la fabrication de produits laitiers, comme additif d'aliments pour animaux ou pour le travail des matières premières textiles.

**10.** Procédé de production d'un polypeptide ayant une activité de phospholipase selon la revendication 7, **caractérisé en ce que** l'on cultive une cellule hôte selon l'une des revendications 4 ou 5 dans des conditions qui favorisent l'expression du polypeptide et que l'on extrait ensuite le polypeptide.

**11.** Procédé de démucilagination de l'huile végétale, **caractérisé en ce que** l'on

a) traite préalablement, le cas échéant, l'huile végétale à traiter,
b) ajoute à l'huile le cas échéant ainsi préalablement traitée une solution aqueuse contenant un polypeptide selon la revendication 7 ou une composition de phospholipase selon la revendication 8,
c) chauffe la préparation réactionnelle selon b) 1 à 12 h à une température située entre 30° et 70 °C, et
d) sépare la phase aqueuse et la phase huileuse l'une de l'autre.

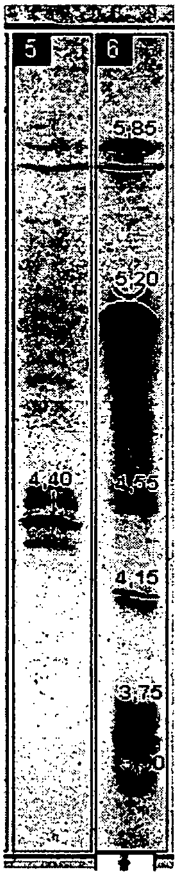


Figur 1

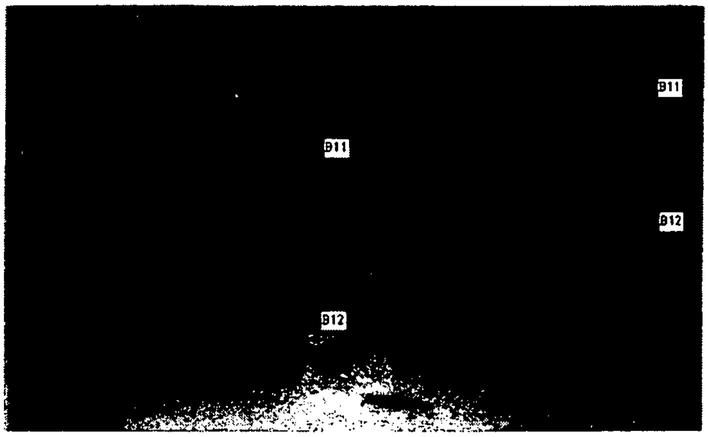


Figur 2

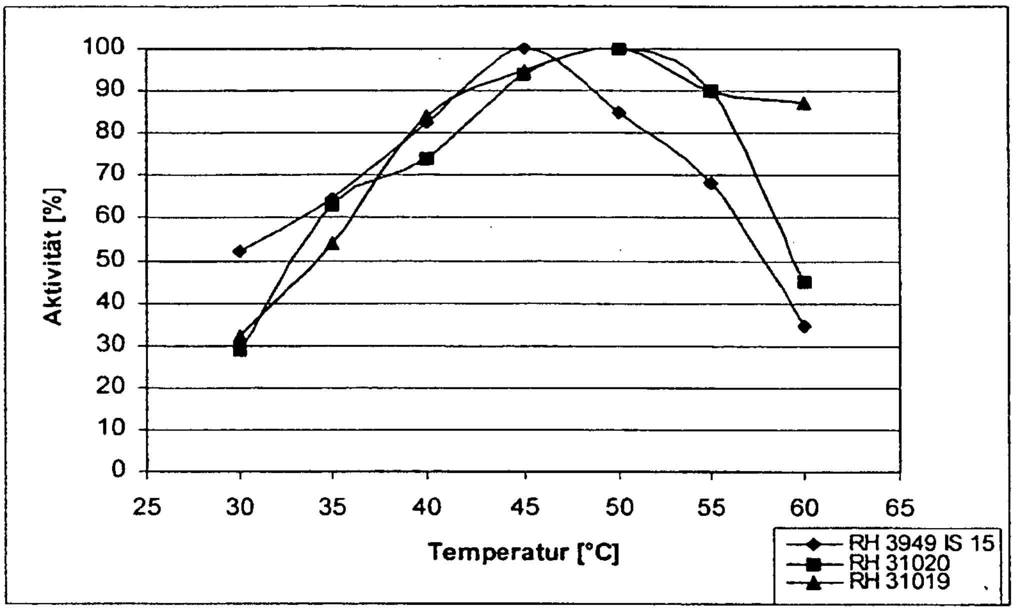
100
90
80
70
60
50
40
30
20
10
0
Aktivität [%]
25
30
35
40
45
50
55
60
65
Temperatur [°C]
RH 3949 IS 15
RH 31020
RH 31019

Figur 3

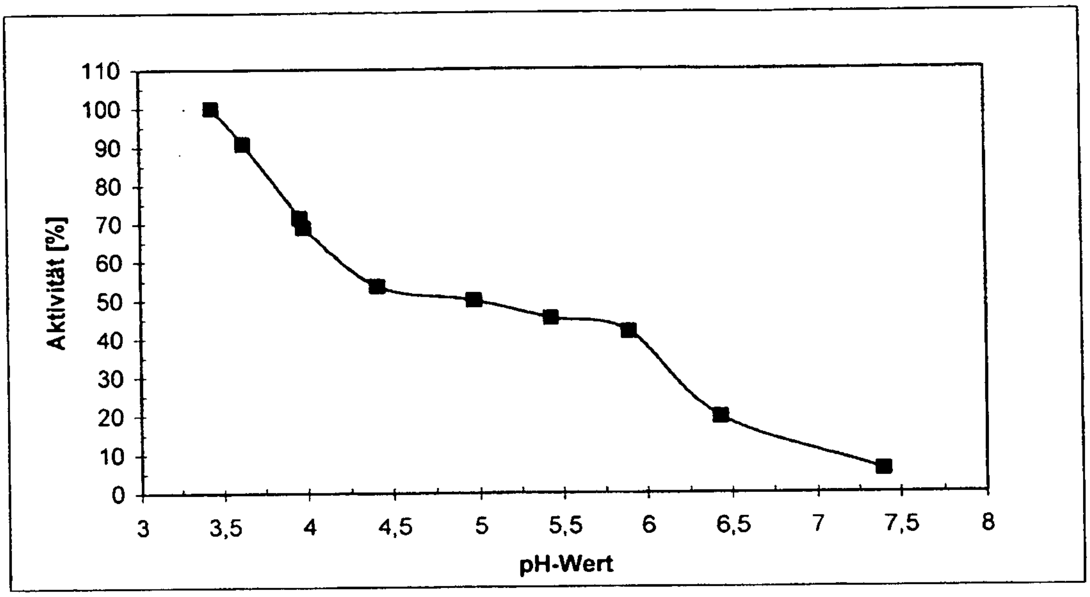
110
100
90
80
70
60
50
40
30
20
10
0
Aktivität [%]
3
3,5
4
4,5
5
5,5
6
6,5
7
7,5
8
pH-Wert

Figur 4

```
1     AAGCTTCTCC ACCATCATAT TCATGCTTTT CAGCCCTTTC AGCAATGTGG
51    TCCGCGGTTC AAACTACGAA TGCTCCAATG GCAATCACCT ATCTATCCTT
101   CGCGAGGGAT GAGACCAAAT CACATTGTTT CAATCTCCCA AGACTTTGGC
151   ATGCTTGGCC TTACTGCTGA TCCACCGTCC CAATATGAGA ACCCCTGGCT
201   AAGGGACACC GCCCCATTTA TTCAAATACC GAATGATGGC TGCCTCACAT
251   TGGGGTTGGG TAGAGAGAGC GATATTTGAT CTTATTGTCC CCTCTAGCTG
301   AATCTTCACG CGGATTATAG CGTGAGGTGG CCTCATACGA CCCAAGATGA
                                                       m
351   AGTCCATCGC AGTGGCGTGC GCTGTCGCCG GCCTATTATT GTCCGGTAGG
       k  s  i   a  v  a  c  a  v  a   g  l  l   l  s
401   TGAATCGTTC TGCCTTGAAG TGTGGTTCAA ACTAGTCAAA TCCGCCTGCG
451   AAACTGGTAC TGATGCCGTC GGACTTCAAT AGGTGCGAGT GGTGCTCCAG
                                         g  a  s   g  a  p
501   AGCCCTTTCA TGGTGAAATC CTACAGCGTG CCCTGCCCAA TGCCCCCGAT
       e  p  f   h  g  e  i  l  q  r   a  l  p   n  a  p  d
551   GGATACACAC CCAGTACAGT CGGTTGTCCT GCCAGTCGCC CTACCATTCG
       g  y  t   p  s  t   v  q  c  p   a  s  r   p  t  i
601   CAGTGCCGCA AAGTTGTCGC CCAACGAGAC GTCATGGCTT CAGACGCGTC
       r  s  a  a  k  l  s   p  n  e   t  s  w  l   q  t  r
651   GAGGCAAGAC TACTTCTGCG ATGAAGGACT TCTTTAGTCA TGTCAAGATT
       r  g  k   t  t  s  a   m  k  d   f  f  s   h  v  k  i
701   CAAGACTTCG ACGCGGTGGG GTACATTGAC CGCCATTCCA GTAACTCGTC
       q  d  f   d  a  v   g  y  i  d   r  h  s   s  n  s
751   GGATCTTCCC AATATCGGCA TCGCAATCTC TGGTGGAGGT TATCGAGCAT
       s  d  l  p  n  i  g   i  a  i   s  g  g  g   y  r  a
801   TGATGAACGG CGCAGGTGCG ATTAAGGCTT TTGATAGTCG TACGCCGAAT
       l  m  n   g  a  g  a   i  k  a   f  d  s   r  t  p  n
851   TCTACGAGCC CCGGTCAGTT GGGTGGATTG CTGCAGTCAG CCACTTATCT
       s  t  s   p  g  q   l  g  g  l   l  q  s   a  t  y
901   TTCTGGCCTG AGTGGTGGAT CATGGCTCGT TGGCTCAATC TACATCAACA
       l  s  g  l  s  g  g   s  w  l   v  g  s  i   y  i  n
951   ACTTTACTAC TATCTCTGCG CTGCAGACAC ACCAAAAGGG CACCGTTTGG
       n  f  t   t  i  s  a   l  q  t   h  q  k   g  t  v  w
1001  CAATTTCAGA ATTCAATATT CGAAGGTCCC GATGGGGGCA GCATTCAGAT
       q  f  q   n  s  i   f  e  g  p   d  g  g   s  i  q
1051  TTTGGATTCA GCATCCTATT ATAAGGACAT CAGCAATGCG GTGTCCGGAA
       i  l  d  s  a  s  y   y  k  d   i  s  n  a   v  s  g
1101  AGGCGGATGC GGGCTACCCA ACTTCCATCA CTGACTACTG GTACTGATAG
       k  a  d   a  g  y  p   t  s  i   t  d  y
1151  TCGTCGGTCT TCTTTGTCGC TACATAACAT GCTGATGATT TCACAGGGGC
                                                      w  g
1201  CGTGCTTTGT CCTACCAGCT GATCAATGCA ACCAACGGTG GTCCTAGCTA
       r  a  l   s  y  q   l  i  n  a   t  n  g   g  p  s
1251  TACATGGTCC TCCATTGCGC TAACCGACAC ATTTCAGCAG GCAGAGATGC
       y  t  w  s  s  i  a   l  t  d   t  f  q  q   a  e  m
1301  CGATGCCTGT AGTTGTTGCA GATGGTCGCT ACCCCGGAGA ACTTATTATC
       p  m  p   v  v  v  a   d  g  r   y  p  g   e  l  i  i
1351  AGCAGCAATG CCACCATCTA TGAATTTAAT CCTTGGGAAT TTGGAACCTT
       s  s  n   a  t  i   y  e  f  n   p  w  e   f  g  t
1401  TGACCCCACA GTTTTTGGAT TTGCCCCTCT TGAGTATCTT GGCACCAAAT
       f  d  p  t  v  f  g   f  a  p   l  e  y  l   g  t  k
1451  TCAATGGAGG CTCAGTTCCG AGTAATGAGA GCTGTGTGCG CGGCTTTGAC
       f  n  g   g  s  v  p   s  n  e   s  c  v   r  g  f  d
1501  AATGCGGGCT TCGTCATGGG TACATCCTCT ACTCTCTTCA ATCAGTTCCT
       n  a  g   f  v  m   g  t  s  s   t  l  f   n  q  f
1551  TCTTCAGATC AACTCTACGG CTTTGCCGGA TTGGCTGAAA TCCATCTTCA
       l  l  q  i  n  s  t   a  l  p   d  w  l  k   s  i  f
1601  CGGACATCCT GAGGGACATC GGCGAAAAGG ATGAGGACAT TGCTCTATAC
       t  d  i   l  r  d  i   g  e  k   d  e  d   i  a  l  y
```

```
1651   GCGCCCAACC CATTCTACCA CTATTCCAAC AATACCAACC CCAATGCCCC
         a  p  n   p  f  y   h  y  s  n   n  t  n   p  n  a
1701   TCAATCTGAA CTGGACCTGG TGGACGGTGG TGAAGATCTG CAAAACATAC
       p  q  s  e   l  d  l   v  d  g   g  e  d  l   q  n  i
1751   CGCTGCACCC ATTGATCCAG CCAGAGCGTC ATGTCGATGT TATCTTCGCC
       p  l  h   p  l  i  q   p  e  r   h  v  d   v  i  f  a
1801   GTTGATTCCT CTGCCGATAC CAAGTACAGC TGGCCCAACG GCACTGCCCT
         v  d  s   s  a  d   t  k  y  s   w  p  n   g  t  a
1851   TGTTGCTACT TATGAGCGTA GCCTGAACAC ATCAGGCATC GCTAATGGCA
       l  v  a  t   y  e  r   s  l  n   t  s  g  i   a  n  g
1901   CCTCCTTTCC TGCAATTCCC GATCAGGATA CGTTCGTGAA CGAAGGCCTG
         t  s  f   p  a  i  p   d  q  d   t  f  v   n  e  g  l
1951   AACACTCGAC CCACGTTCTT CGGGTGCAAC AGCTCAAACA TGACGGGCCC
         n  t  r   p  t  f   f  g  c  n   s  s  n   m  t  g
2001   ATCGCCCTTG ATTGTATATC TCCCAAACTA TCCCTACACC GCTTACTCCA
       p  s  p  l   i  v  y   l  p  n   y  p  y  t   a  y  s
2051   ACTTTTCTAC CTTCCAGCCA GACTACACAG AAGAAGAGCG AGATGCTACC
         n  f  s   t  f  q  p   d  y  t   e  e  e   r  d  a  t
2101   ATCCTCAACG GATATGATGT GGTGACAATG GGTAACAGCA CTCGTGATGG
         i  l  n   g  y  d   v  v  t  m   g  n  s   t  r  d
2151   CAACTGGTCA ACCTGCGTTG GCTGTGCCAT CTTGAGTCGG TCTTTCGAAC
       g  n  w  s   t  c  v   g  c  a   i  l  s  r   s  f  e
2201   GCACAAACAC TAATGTGCCG GAAATCTGCA AACAATGTTT CCAGAGGTAT
         r  t  n   t  n  v  p   e  i  c   k  q  c   f  q  r  y
2251   TGCTGGGACG GCTCTATCAA CAACACCACT CCTGCGGTTT ACGAACCGGT
         c  w  d   g  s  i   n  n  t  t   p  a  v   y  e  p
2301   CACGATTTTG GATAGCGCAG GCTCCGGGAT CTTTCCAAGT ATTCTCGCTG
       v  t  i  l   d  s  a   g  s  g   i  f  p  s   i  l  a
2351   CTGCAATGGC TGCTATTGTT GCCTCTTGGA CTATTCTATA GAATTCATTT
         a  a  m   a  a  i  v   a  s  w   t  i  l   -
2401   CGAGAGTTTC GCGAAATGTC TATTTCGGCC TGATTCTATG CTGACTGAGC
2451   TGTATCTACC CGTCACAACT TTTGTCAGAA GCCATGTTTG TCCATTTGGA
2501   AATTTGACGA GCAATATTGT CGTTGGATCT ATCTATCTAT CGCTTTGTAT
2551   CCCTCTTGTA TATAGCTTAT GCACGAAAAT AAAATATCAT GGCCATGACA
2601   TCCCTTCAGG CGCAATCAAT TACATATAAG CTGGGGGTCA TTAAAATGCC
2651   ACGTGACGGT GGGGTCCGAG TGTTGCTATG ACAACATCCA CGTGACTTCT
2701   CAAACAAGAA ACTTAAGCAC AAACGCCGCA GCTCTAGCGG GCGGCCAAAC
2751   GCAACAACAA CACATATCTA ATCAACAAGC TAGGTCTTCT TAAGCCACAG
2801   CAAAGCCCCT GCTTGAAAGC TT
```

Figur 5

```
   1 AAGCTTCTCC ACCATCATAT TCATGCTTTT CAGCCCTTTC AGCAATGTGG
  51 TCCGCGGTTC AAACTACGAA TGCTCCAATG GCAATCACCT ATCTATCCTT
 101 CGCGAGGGAT GAGACCAAAT CACATTGTTT CAATCTCCCA AGACTTTGGC
 151 ATGCTTGGCC TTACTGCTGA TCCACCGTCC CAATATGAGA ACCCCTGGCT
 201 AAGGGACACC GCCCCATTTA TTCAAATACC GAATGATGGC TGCCTCACAT
 251 TGGGGTTGGG TAGAGAGAGC GATATTTGAT CTTATTGTCC CCTCTAGCTG
 301 AATCTTCACG CGGATTATAG CGTGAGGTGG CCTCATACGA CCCAAGATGA
 351 AGTCCATCGC AGTGGCGTGC GCTGTCGCCG GCCTATTATT GTCCGGTAGG
 401 TGAATCGTTC TGCCTTGAAG TGTGGTTCAA ACTAGTCAAA TCCGCCTGCG
 451 AAACTGGTAC TGATGCCGTC GGACTTCAAT AGGTGCGAGT GGTGCTCCAG
 501 AGCCCTTTCA TGGTGAAATC CTACAGCGTG CCCTGCCCAA TGCCCCCGAT
 551 GGATACACAC CCAGTACAGT CGGTTGTCCT GCCAGTCGCC CTACCATTCG
 601 CAGTGCCGCA AAGTTGTCGC CCAACGAGAC GTCATGGCTT CAGACGCGTC
 651 GAGGCAAGAC TACTTCTGCG ATGAAGGACT TCTTTAGTCA TGTCAAGATT
 701 CAAGACTTCG ACGCGGTGGG GTACATTGAC CGCCATTCCA GTAACTCGTC
 751 GGATCTTCCC AATATCGGCA TCGCAATCTC TGGTGGAGGT TATCGAGCAT
 801 TGATGAACGG CGCAGGTGCG ATTAAGGCTT TTGATAGTCG TACGCCGAAT
 851 TCTACGAGCC CCGGTCAGTT GGGTGGATTG CTGCAGTCAG CCACTTATCT
 901 TTCTGGCCTG AGTGGTGGAT CATGGCTCGT TGGCTCAATC TACATCAACA
 951 ACTTTACTAC TATCTCTGCG CTGCAGACAC ACCAAAAGGG CACCGTTTGG
1001 CAATTTCAGA ATTCAATATT CGAAGGTCCC GATGGGGGCA GCATTCAGAT
1051 TTTGGATTCA GCATCCTATT ATAAGGACAT CAGCAATGCG GTGTCCGGAA
1101 AGGCGGATGC GGGCTACCCA ACTTCCATCA CTGACTACTG GTACTGATAG
1151 TCGTCGGTCT TCTTTGTCGC TACATAACAT GCTGATGATT TCACAGGGGC
1201 CGTGCTTTGT CCTACCAGCT GATCAATGCA ACCAACGGTG GTCCTAGCTA
1251 TACATGGTCC TCCATTGCGC TAACCGACAC ATTTCAGCAG GCAGAGATGC
1301 CGATGCCTGT AGTTGTTGCA GATGGTCGCT ACCCCGGAGA ACTTATTATC
1351 AGCAGCAATG CCACCATCTA TGAATTTAAT CCTTGGGAAT TTGGAACCTT
1401 TGACCCCACA GTTTTTGGAT TTGCCCCTCT TGAGTATCTT GGCACCAAAT
1451 TCAATGGAGG CTCAGTTCCG AGTAATGAGA GCTGTGTGCG CGGCTTTGAC
1501 AATGCGGGCT TCGTCATGGG TACATCCTCT ACTCTCTTCA ATCAGTTCCT
1551 TCTTCAGATC AACTCTACGG CTTTGCCGGA TTGGCTGAAA TCCATCTTCA
1601 CGGACATCCT GAGGGACATC GGCGAAAAGG ATGAGGACAT TGCTCTATAC
1651 GCGCCCAACC CATTCTACCA CTATTCCAAC AATACCAACC CCAATGCCCC
1701 TCAATCTGAA CTGGACCTGG TGGACGGTGG TGAAGATCTG CAAAACATAC
1751 CGCTGCACCC ATTGATCCAG CCAGAGCGTC ATGTCGATGT TATCTTCGCC
1801 GTTGATTCCT CTGCCGATAC CAAGTACAGC TGGCCCAACG GCACTGCCCT
1851 TGTTGCTACT TATGAGCGTA GCCTGAACAC ATCAGGCATC GCTAATGGCA
1901 CCTCCTTTCC TGCAATTCCC GATCAGGATA CGTTCGTGAA CGAAGGCCTG
1951 AACACTCGAC CCACGTTCTT CGGGTGCAAC AGCTCAAACA TGACGGGCCC
2001 ATCGCCCTTG ATTGTATATC TCCCAAACTA TCCCTACACC GCTTACTCCA
2051 ACTTTTCTAC CTTCCAGCCA GACTACACAG AAGAAGAGCG AGATGCTACC
2101 ATCCTCAACG GATATGATGT GGTGACAATG GGTAACAGCA CTCGTGATGG
2151 CAACTGGTCA ACCTGCGTTG GCTGTGCCAT CTTGAGTCGG TCTTTCGAAC
2201 GCACAAACAC TAATGTGCCG GAAATCTGCA AACAATGTTT CCAGAGGTAT
2251 TGCTGGGACG GCTCTATCAA CAACACCACT CCTGCGGTTT ACGAACCGGT
2301 CACGATTTTG GATAGCGCAG GCTCCGGGAT CTTTCCAAGT ATTCTCGCTG
2351 CTGCAATGGC TGCTATTGTT GCCTCTTGGA CTATTCTATA GAATTCATTT
2401 CGAGAGTTTC GCGAAATGTC TATTTCGGCC TGATTCTATG CTGACTGAGC
2451 TGTATCTACC CGTCACAACT TTTGTCAGAA GCCATGTTTG TCCATTTGGA
2501 AATTTGACGA GCAATATTGT CGTTGGATCT ATCTATCTAT CGCTTTGTAT
2551 CCCTCTTGTA TATAGCTTAT GCACGAAAAT AAAATATCAT GGCCATGACA
2601 TCCCTTCAGG CGCAATCAAT TACATATAAG CTGGGGGTCA TTAAAATGCC
2651 ACGTGACGGT GGGGTCCGAG TGTTGCTATG ACAACATCCA CGTGACTTCT
2701 CAAACAAGAA ACTTAAGCAC AAACGCCGCA GCTCTAGCGG GCGGCCAAAC
2751 GCAACAACAA CACATATCTA ATCAACAAGC TAGGTCTTCT TAAGCCACAG
2801 CAAAGCCCCT GCTTGAAAGC TT
```

Figur 6

| | | | | | |
|---|---|---|---|---|---|
| 1 | MKSIAVACAV | AGLLLSGASG | APEPFHGEIL | QRALPNAPDG | YTPSTVGCPA |
| 51 | SRPTIRSAAK | LSPNETSWLQ | TRRGKTTSAM | KDFFSHVKIQ | DFDAVGYIDR |
| 101 | HSSNSSDLPN | IGIAISGGGY | RALMNGAGAI | KAFDSRTPNS | TSPGQLGGLL |
| 151 | QSATYLSGLS | GGSWLVGSIY | INNFTTISAL | QTHQKGTVWQ | FQNSIFEGPD |
| 201 | GGSIQILDSA | SYYKDISNAV | SGKADAGYPT | SITDYWGRAL | SYQLINATNG |
| 251 | GPSYTWSSIA | LTDTFQQAEM | PMPVVVADGR | YPGELIISSN | ATIYEFNPWE |
| 301 | FGTFDPTVFG | FAPLEYLGTK | FNGGSVPSNE | SCVRGFDNAG | FVMGTSSTLF |
| 351 | NQFLLQINST | ALPDWLKSIF | TDILRDIGEK | DEDIALYAPN | PFYHYSNNTN |
| 401 | PNAPQSELDL | VDGGEDLQNI | PLHPLIQPER | HVDVIFAVDS | SADTKYSWPN |
| 451 | GTALVATYER | SLNTSGIANG | TSFPAIPDQD | TFVNEGLNTR | PTFFGCNSSN |
| 501 | MTGPSPLIVY | LPNYPYTAYS | NFSTFQPDYT | EEERDATILN | GYDVVTMGNS |
| 551 | TRDGNWSTCV | GCAILSRSFE | RTNTNVPEIC | KQCFQRYCWD | GSINNTTPAV |
| 601 | YEPVTILDSA | GSGIFPSILA | AAMAAIVASW | TIL | |

Figur 7

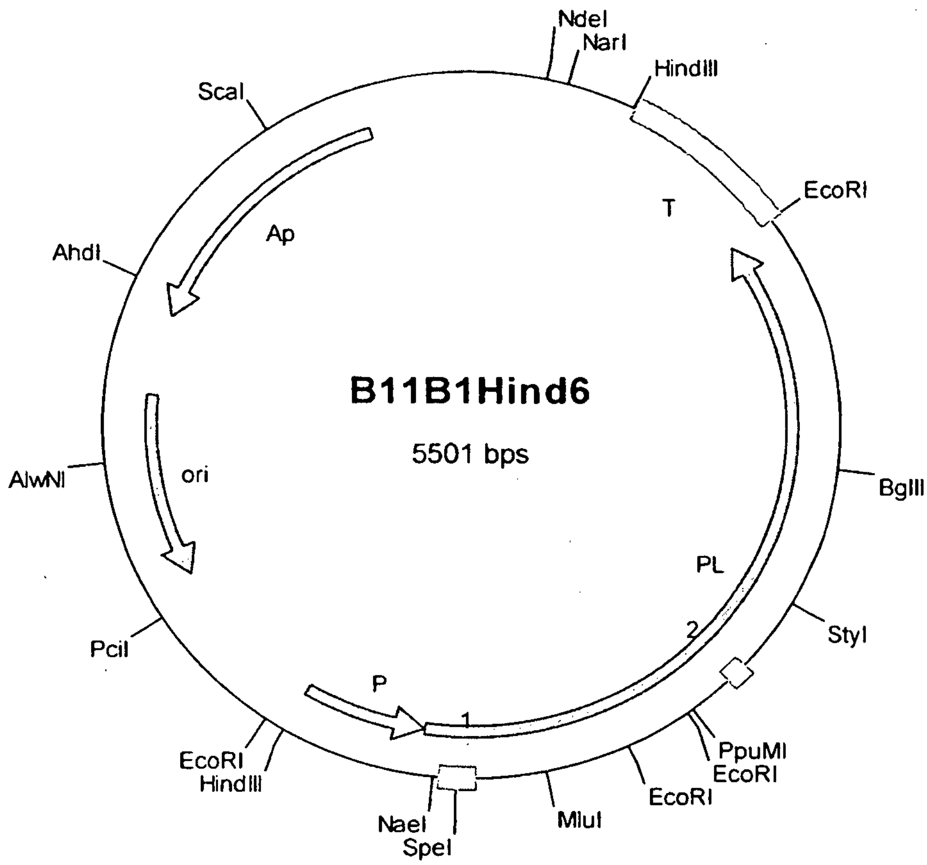
NdeI
NarI
HindIII
EcoRI
ScaI
T
Ap
AhdI
B11B1Hind6
5501 bps
AlwNI
ori
BglII
PL
PciI
2
StyI
P
1
PpuMI
EcoRI
EcoRI
HindIII
EcoRI
MluI
NaeI
SpeI

Figur 8

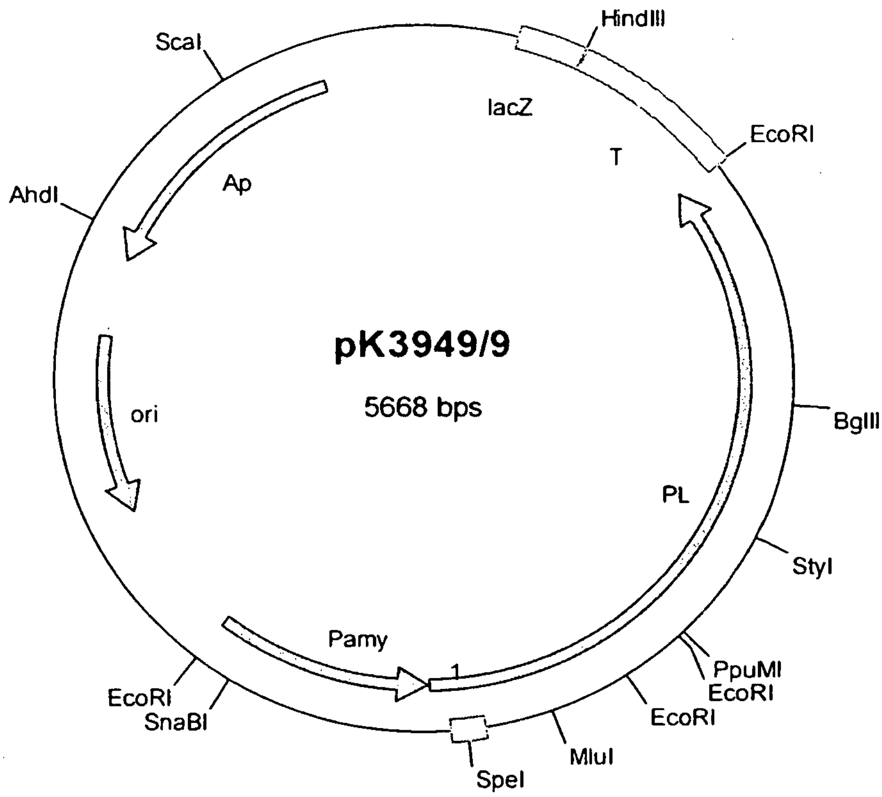
pK3949/9
5668 bps
HindIII
lacZ
T
EcoRI
ScaI
Ap
AhdI
ori
BglII
PL
StyI
Pamy
1
PpuMI
EcoRI
EcoRI
EcoRI
SnaBI
MluI
SpeI

Figur 9

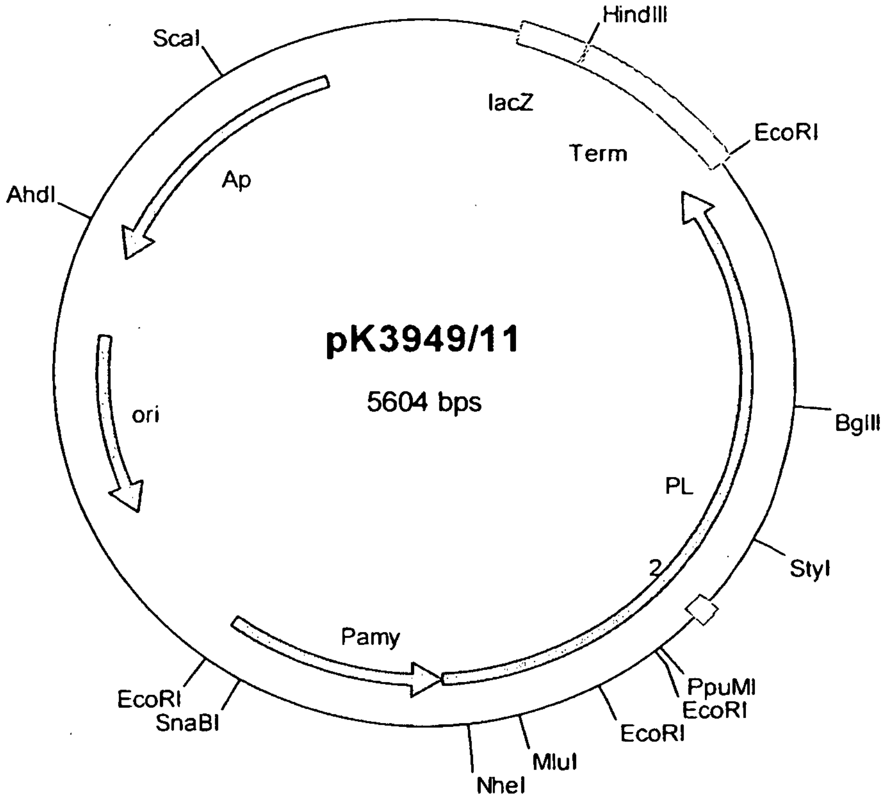
HindIII
ScaI
lacZ
EcoRI
Term
Ap
AhdI
pK3949/11
5604 bps
ori
BglII
PL
2
StyI
Pamy
PpuMI
EcoRI
EcoRI
SnaBI
EcoRI
MluI
NheI

**Figur 10**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9831790 AB
- WO 9826057 A
- JP 10155493 A
- WO 0224881 A
- JP 03151879 B
- EP 0575133 A2
- US 5538874 A
- US 5378623 A
- US 5521080 A
- WO 9818912 A
- WO 03097825 A
- WO 0127251 A
- WO 0129222 A
- WO 0028044 A
- EP 0808903 A
- WO 2004097012 A
- WO 0032758 A
- WO 03060112 A
- WO 2004111216 A
- WO 02066622 A
- EP 0513709 B2
- DE 339556 A4
- JP 7177884 A
- EP 99973065 A
- WO 9831790 A
- US 6759225 B
- WO 01027251 A
- US 4873192 A
- WO 9500636 A
- WO 9114772 A
- EP 07818346 A
- DE 10200610046719


### In der Beschreibung aufgeführte Nicht-Patentliteratur


- *FEMS Microbiol. Let.,* 1983, vol. 18, 15-18
- *Annu. Rev. Biochem.,* 1972, vol. 41, 129-160
- *Biochemistry,* 04. Mai 1999, vol. 38 (18), 5864-5871
- **SHEN et al.** *FEMS Microbiol Lett.,* 2004, vol. 239 (1), 87-93
- **N. MASUDA et al.** *Eur. J. Biochem.,* 1991, vol. 202, 783-787
- **LEE et al.** *J. Biol. Chem.,* 1994, vol. 269, 19725-19730
- **MERKEL et al.** *J. Biol. Chem.,* 1999, vol. 274, 28121-28127
- **WATANABE et al.** *FEMS Microbiology Letters,* 1994, vol. 124, 29-34
- **OISHI et al.** *Biosci. Biotechnol. Biochem.,* 1999, vol. 63, 83-90
- *J. Biol. Chem.,* 1998, vol. 273 (40), 26078-26086
- *Medical Mycology,* 1998, vol. 37, 61-67
- **SHEN et al.** *FEMS Microbiol. Letters,* 2004, vol. 239 (1), 87-93
- **MYERS, E.W. ; W. MILLER.** *Commun Assoc Comput Mach,* 1988, vol. 18, 341-343
- *CABIOS,* vol. 1, 11-17
- **CHAO, K-M ; W.R. PEARSON ; W. MILLER.** Aligning two sequences within a specified diagonal band. *CABIOS,* 1992, vol. 5, 481-487
- **KUNKEL.** *Proc. Natl. Acad. Sci. USA,* 1985, vol. 82, 488
- **KUNKEL et al.** *Methods in Enzymol.,* 1987, vol. 154, 367
- Techniques in Molecular Biology. Mac Millan Publishing Company, 1983
- **MODEL VON DAYHOFF et al.** Atlas of Protein Sequence and Structure. Natl. Biomed. Res. Found, 1978
- **B. SAMBROOK et al.** Molecular Cloning: A Laboratory Manual. Cold Spring Harbor Laboratory Press, 1989
- **PENTTILÄ et al.** *Gene,* 1987, vol. 61, 155-164
- **VON GROSS.** The Cyanogen Bromide Reaction. *Methods Enzymol,* 1967, vol. XI, 238-255
- **SCHÄGGER ; JAGOW.** *Anal. Biochem.,* 1987, vol. 199, 223-231
- **MATSUDAIRA.** *J. Biol. Chem.,* 1987, vol. 262, 10035-10038
- **VON HYNES, M.J et al.** *Mol. Cell. Biol.,* 1983, vol. 3, 1430-1439
- **NAKAI ; KANEHISA.** *Genomics,* 1992, vol. 14, 897-911
- **WIRSEL et al.** *Mol. Microbiol.,* 1989, vol. 3 (1), 3-14
- **VON YELTON et al.** *Proc. Natl. Acad. Sci. USA,* 1984, vol. 81, 1470-1474
- **PUNT et al.** *Gene,* 1987, vol. 56, 117-124

SEQUENZPROTOKOLL

<110> AB ENZYMES GMBH

<120> KLONIERUNG, EXPRESSION UND VERWENDUNG SAURER PHOSPHOLIPASEN

<130> 16696EP

<140> EP 07 818 346.4
<141> 2007-09-21

<150> DE 10 2006 100 46 719.1
<151> 2006-10-02

<160> 25

<170> PatentIn version 3.1

<210> 1
<211> 2822
<212> DNA
<213> Aspergillus fumigatus

<220>
<221> CDS
<222> (347)..(395)
<223>

<220>
<221> CDS
<222> (483)..(1140)
<223>

<220>
<221> CDS
<222> (1197)..(2388)
<223>

<400> 1

```
aagcttctcc accatcatat tcatgctttt cagccctttc agcaatgtgg tccgcggttc      60

aaactacgaa tgctccaatg gcaatcacct atctatcctt cgcgagggat gagaccaaat     120

cacattgttt caatctccca agactttggc atgcttggcc ttactgctga tccaccgtcc     180

caatatgaga acccctggct aagggacacc gccccattta ttcaaatacc gaatgatggc     240

tgcctcacat tggggttggg tagagagagc gatatttgat cttattgtcc cctctagctg     300

aatcttcacg cggattatag cgtgaggtgg cctcatacga cccaag atg aag tcc        355
                                                   Met Lys Ser
                                                   1

atc gca gtg gcg tgc gct gtc gcc ggc cta tta ttg tcc g gtaggtgaat      405
Ile Ala Val Ala Cys Ala Val Ala Gly Leu Leu Leu Ser
    5                   10                  15

cgttctgcct tgaagtgtgg ttcaaactag tcaaatccgc ctgcgaaact ggtactgatg     465

ccgtcggact tcaatag gt  gcg agt ggt gct cca gag ccc ttt cat ggt        514
                   Gly Ala Ser Gly Ala Pro Glu Pro Phe His Gly
                               20                  25

gaa atc cta cag cgt gcc ctg ccc aat gcc ccc gat gga tac aca ccc       562
Glu Ile Leu Gln Arg Ala Leu Pro Asn Ala Pro Asp Gly Tyr Thr Pro
        30                  35                  40

agt aca gtc ggt tgt cct gcc agt cgc cct acc att cgc agt gcc gca       610
Ser Thr Val Gly Cys Pro Ala Ser Arg Pro Thr Ile Arg Ser Ala Ala
    45                  50                  55

aag ttg tcg ccc aac gag acg tca tgg ctt cag acg cgt cga ggc aag       658
Lys Leu Ser Pro Asn Glu Thr Ser Trp Leu Gln Thr Arg Arg Gly Lys
60                  65                  70                  75

act act tct gcg atg aag gac ttc ttt agt cat gtc aag att caa gac       706
Thr Thr Ser Ala Met Lys Asp Phe Phe Ser His Val Lys Ile Gln Asp
                80                  85                  90

ttc gac gcg gtg ggg tac att gac cgc cat tcc agt aac tcg tcg gat       754
Phe Asp Ala Val Gly Tyr Ile Asp Arg His Ser Ser Asn Ser Ser Asp
            95                  100                 105
```

```
ctt ccc aat atc ggc atc gca atc tct ggt gga ggt tat cga gca ttg       802
Leu Pro Asn Ile Gly Ile Ala Ile Ser Gly Gly Gly Tyr Arg Ala Leu
        110                 115                 120

atg aac ggc gca ggt gcg att aag gct ttt gat agt cgt acg ccg aat       850
Met Asn Gly Ala Gly Ala Ile Lys Ala Phe Asp Ser Arg Thr Pro Asn
    125                 130                 135

tct acg agc ccc ggt cag ttg ggt gga ttg ctg cag tca gcc act tat       898
Ser Thr Ser Pro Gly Gln Leu Gly Gly Leu Leu Gln Ser Ala Thr Tyr
140                 145                 150                 155

ctt tct ggc ctg agt ggt gga tca tgg ctc gtt ggc tca atc tac atc       946
Leu Ser Gly Leu Ser Gly Gly Ser Trp Leu Val Gly Ser Ile Tyr Ile
                160                 165                 170

aac aac ttt act act atc tct gcg ctg cag aca cac caa aag ggc acc       994
Asn Asn Phe Thr Thr Ile Ser Ala Leu Gln Thr His Gln Lys Gly Thr
            175                 180                 185

gtt tgg caa ttt cag aat tca ata ttc gaa ggt ccc gat ggg ggc agc      1042
Val Trp Gln Phe Gln Asn Ser Ile Phe Glu Gly Pro Asp Gly Gly Ser
        190                 195                 200

att cag att ttg gat tca gca tcc tat tat aag gac atc agc aat gcg      1090
Ile Gln Ile Leu Asp Ser Ala Ser Tyr Tyr Lys Asp Ile Ser Asn Ala
    205                 210                 215

gtg tcc gga aag gcg gat gcg ggc tac cca act tcc atc act gac tac      1138
Val Ser Gly Lys Ala Asp Ala Gly Tyr Pro Thr Ser Ile Thr Asp Tyr
220                 225                 230                 235

tg  gtactgatag tcgtcggtct tctttgtcgc tacataacat gctgatgatt tcacag   1196
Trp

g ggc cgt gct ttg tcc tac cag ctg atc aat gca acc aac ggt ggt cct    1245
  Gly Arg Ala Leu Ser Tyr Gln Leu Ile Asn Ala Thr Asn Gly Gly Pro
              240                 245                 250

agc tat aca tgg tcc tcc att gcg cta acc gac aca ttt cag cag gca      1293
Ser Tyr Thr Trp Ser Ser Ile Ala Leu Thr Asp Thr Phe Gln Gln Ala
        255                 260                 265

gag atg ccg atg cct gta gtt gtt gca gat ggt cgc tac ccc gga gaa      1341
Glu Met Pro Met Pro Val Val Val Ala Asp Gly Arg Tyr Pro Gly Glu
    270                 275                 280

ctt att atc agc agc aat gcc acc atc tat gaa ttt aat cct tgg gaa      1389
Leu Ile Ile Ser Ser Asn Ala Thr Ile Tyr Glu Phe Asn Pro Trp Glu
285                 290                 295                 300

ttt gga acc ttt gac ccc aca gtt ttt gga ttt gcc cct ctt gag tat      1437
Phe Gly Thr Phe Asp Pro Thr Val Phe Gly Phe Ala Pro Leu Glu Tyr
                305                 310                 315

ctt ggc acc aaa ttc aat gga ggc tca gtt ccg agt aat gag agc tgt      1485
Leu Gly Thr Lys Phe Asn Gly Gly Ser Val Pro Ser Asn Glu Ser Cys
            320                 325                 330
```

```
gtg cgc ggc ttt gac aat gcg ggc ttc gtc atg ggt aca tcc tct act      1533
Val Arg Gly Phe Asp Asn Ala Gly Phe Val Met Gly Thr Ser Ser Thr
        335                 340                 345

ctc ttc aat cag ttc ctt ctt cag atc aac tct acg gct ttg ccg gat      1581
Leu Phe Asn Gln Phe Leu Leu Gln Ile Asn Ser Thr Ala Leu Pro Asp
    350                 355                 360

tgg ctg aaa tcc atc ttc acg gac atc ctg agg gac atc ggc gaa aag      1629
Trp Leu Lys Ser Ile Phe Thr Asp Ile Leu Arg Asp Ile Gly Glu Lys
365                 370                 375                 380

gat gag gac att gct cta tac gcg ccc aac cca ttc tac cac tat tcc      1677
Asp Glu Asp Ile Ala Leu Tyr Ala Pro Asn Pro Phe Tyr His Tyr Ser
                385                 390                 395

aac aat acc aac ccc aat gcc cct caa tct gaa ctg gac ctg gtg gac      1725
Asn Asn Thr Asn Pro Asn Ala Pro Gln Ser Glu Leu Asp Leu Val Asp
            400                 405                 410

ggt ggt gaa gat ctg caa aac ata ccg ctg cac cca ttg atc cag cca      1773
Gly Gly Glu Asp Leu Gln Asn Ile Pro Leu His Pro Leu Ile Gln Pro
        415                 420                 425

gag cgt cat gtc gat gtt atc ttc gcc gtt gat tcc tct gcc gat acc      1821
Glu Arg His Val Asp Val Ile Phe Ala Val Asp Ser Ser Ala Asp Thr
    430                 435                 440

aag tac agc tgg ccc aac ggc act gcc ctt gtt gct act tat gag cgt      1869
Lys Tyr Ser Trp Pro Asn Gly Thr Ala Leu Val Ala Thr Tyr Glu Arg
445                 450                 455                 460

agc ctg aac aca tca ggc atc gct aat ggc acc tcc ttt cct gca att      1917
Ser Leu Asn Thr Ser Gly Ile Ala Asn Gly Thr Ser Phe Pro Ala Ile
                465                 470                 475

ccc gat cag gat acg ttc gtg aac gaa ggc ctg aac act cga ccc acg      1965
Pro Asp Gln Asp Thr Phe Val Asn Glu Gly Leu Asn Thr Arg Pro Thr
            480                 485                 490

ttc ttc ggg tgc aac agc tca aac atg acg ggc cca tcg ccc ttg att      2013
Phe Phe Gly Cys Asn Ser Ser Asn Met Thr Gly Pro Ser Pro Leu Ile
        495                 500                 505

gta tat ctc cca aac tat ccc tac acc gct tac tcc aac ttt tct acc      2061
Val Tyr Leu Pro Asn Tyr Pro Tyr Thr Ala Tyr Ser Asn Phe Ser Thr
    510                 515                 520

ttc cag cca gac tac aca gaa gaa gag cga gat gct acc atc ctc aac      2109
Phe Gln Pro Asp Tyr Thr Glu Glu Glu Arg Asp Ala Thr Ile Leu Asn
525                 530                 535                 540

gga tat gat gtg gtg aca atg ggt aac agc act cgt gat ggc aac tgg      2157
Gly Tyr Asp Val Val Thr Met Gly Asn Ser Thr Arg Asp Gly Asn Trp
                545                 550                 555

tca acc tgc gtt ggc tgt gcc atc ttg agt cgg tct ttc gaa cgc aca      2205
Ser Thr Cys Val Gly Cys Ala Ile Leu Ser Arg Ser Phe Glu Arg Thr
            560                 565                 570
```

```
aac act aat gtg ccg gaa atc tgc aaa caa tgt ttc cag agg tat tgc      2253
Asn Thr Asn Val Pro Glu Ile Cys Lys Gln Cys Phe Gln Arg Tyr Cys
        575                 580                 585

tgg gac ggc tct atc aac aac acc act cct gcg gtt tac gaa ccg gtc      2301
Trp Asp Gly Ser Ile Asn Asn Thr Thr Pro Ala Val Tyr Glu Pro Val
    590                 595                 600

acg att ttg gat agc gca ggc tcc ggg atc ttt cca agt att ctc gct      2349
Thr Ile Leu Asp Ser Ala Gly Ser Gly Ile Phe Pro Ser Ile Leu Ala
605                 610                 615                 620

gct gca atg gct gct att gtt gcc tct tgg act att cta tagaattcat       2398
Ala Ala Met Ala Ala Ile Val Ala Ser Trp Thr Ile Leu
                625                 630

ttcgagagtt tcgcgaaatg tctatttcgg cctgattcta tgctgactga gctgtatcta    2458

cccgtcacaa cttttgtcag aagccatgtt tgtccatttg gaaatttgac gagcaatatt    2518

gtcgttggat ctatctatct atcgctttgt atccctcttg tatatagctt atgcacgaaa    2578

ataaaatatc atggccatga catcccttca ggcgcaatca attacatata agctggggt     2638

cattaaaatg ccacgtgacg gtggggtccg agtgttgcta tgacaacatc cacgtgactt    2698

ctcaaacaag aaacttaagc acaaacgccg cagctctagc gggcggccaa acgcaacaac    2758

aacacatatc taatcaacaa gctaggtctt cttaagccac agcaaagccc ctgcttgaaa    2818

gctt                                                                 2822
```

<210> 2
<211> 633
<212> PRT
<213> Aspergillus fumigatus

<400> 2

```
Met Lys Ser Ile Ala Val Ala Cys Ala Val Ala Gly Leu Leu Leu Ser
1               5                   10                  15

Gly Ala Ser Gly Ala Pro Glu Pro Phe His Gly Glu Ile Leu Gln Arg
            20                  25                  30

Ala Leu Pro Asn Ala Pro Asp Gly Tyr Thr Pro Ser Thr Val Gly Cys
        35                  40                  45

Pro Ala Ser Arg Pro Thr Ile Arg Ser Ala Ala Lys Leu Ser Pro Asn
    50                  55                  60

Glu Thr Ser Trp Leu Gln Thr Arg Arg Gly Lys Thr Thr Ser Ala Met
65                  70                  75                  80
```

```
Lys Asp Phe Phe Ser His Val Lys Ile Gln Asp Phe Asp Ala Val Gly
                85                  90                  95

Tyr Ile Asp Arg His Ser Ser Asn Ser Ser Asp Leu Pro Asn Ile Gly
            100                 105                 110

Ile Ala Ile Ser Gly Gly Gly Tyr Arg Ala Leu Met Asn Gly Ala Gly
        115                 120                 125

Ala Ile Lys Ala Phe Asp Ser Arg Thr Pro Asn Ser Thr Ser Pro Gly
    130                 135                 140

Gln Leu Gly Gly Leu Leu Gln Ser Ala Thr Tyr Leu Ser Gly Leu Ser
145                 150                 155                 160

Gly Gly Ser Trp Leu Val Gly Ser Ile Tyr Ile Asn Asn Phe Thr Thr
                165                 170                 175

Ile Ser Ala Leu Gln Thr His Gln Lys Gly Thr Val Trp Gln Phe Gln
            180                 185                 190

Asn Ser Ile Phe Glu Gly Pro Asp Gly Gly Ser Ile Gln Ile Leu Asp
        195                 200                 205

Ser Ala Ser Tyr Tyr Lys Asp Ile Ser Asn Ala Val Ser Gly Lys Ala
    210                 215                 220

Asp Ala Gly Tyr Pro Thr Ser Ile Thr Asp Tyr Trp Gly Arg Ala Leu
225                 230                 235                 240

Ser Tyr Gln Leu Ile Asn Ala Thr Asn Gly Gly Pro Ser Tyr Thr Trp
                245                 250                 255

Ser Ser Ile Ala Leu Thr Asp Thr Phe Gln Gln Ala Glu Met Pro Met
            260                 265                 270

Pro Val Val Val Ala Asp Gly Arg Tyr Pro Gly Glu Leu Ile Ile Ser
        275                 280                 285

Ser Asn Ala Thr Ile Tyr Glu Phe Asn Pro Trp Glu Phe Gly Thr Phe
    290                 295                 300

Asp Pro Thr Val Phe Gly Phe Ala Pro Leu Glu Tyr Leu Gly Thr Lys
305                 310                 315                 320
```

```
Phe Asn Gly Gly Ser Val Pro Ser Asn Glu Ser Cys Val Arg Gly Phe
                325                 330                 335

Asp Asn Ala Gly Phe Val Met Gly Thr Ser Ser Thr Leu Phe Asn Gln
            340                 345                 350

Phe Leu Leu Gln Ile Asn Ser Thr Ala Leu Pro Asp Trp Leu Lys Ser
        355                 360                 365

Ile Phe Thr Asp Ile Leu Arg Asp Ile Gly Glu Lys Asp Glu Asp Ile
    370                 375                 380

Ala Leu Tyr Ala Pro Asn Pro Phe Tyr His Tyr Ser Asn Asn Thr Asn
385                 390                 395                 400

Pro Asn Ala Pro Gln Ser Glu Leu Asp Leu Val Asp Gly Gly Glu Asp
                405                 410                 415

Leu Gln Asn Ile Pro Leu His Pro Leu Ile Gln Pro Glu Arg His Val
            420                 425                 430

Asp Val Ile Phe Ala Val Asp Ser Ser Ala Asp Thr Lys Tyr Ser Trp
        435                 440                 445

Pro Asn Gly Thr Ala Leu Val Ala Thr Tyr Glu Arg Ser Leu Asn Thr
    450                 455                 460

Ser Gly Ile Ala Asn Gly Thr Ser Phe Pro Ala Ile Pro Asp Gln Asp
465                 470                 475                 480

Thr Phe Val Asn Glu Gly Leu Asn Thr Arg Pro Thr Phe Phe Gly Cys
                485                 490                 495

Asn Ser Ser Asn Met Thr Gly Pro Ser Pro Leu Ile Val Tyr Leu Pro
            500                 505                 510

Asn Tyr Pro Tyr Thr Ala Tyr Ser Asn Phe Ser Thr Phe Gln Pro Asp
        515                 520                 525

Tyr Thr Glu Glu Glu Arg Asp Ala Thr Ile Leu Asn Gly Tyr Asp Val
    530                 535                 540

Val Thr Met Gly Asn Ser Thr Arg Asp Gly Asn Trp Ser Thr Cys Val
545                 550                 555                 560

Gly Cys Ala Ile Leu Ser Arg Ser Phe Glu Arg Thr Asn Thr Asn Val
                565                 570                 575
```

Pro Glu Ile Cys Lys Gln Cys Phe Gln Arg Tyr Cys Trp Asp Gly Ser
580 585 590

Ile Asn Asn Thr Thr Pro Ala Val Tyr Glu Pro Val Thr Ile Leu Asp
595 600 605

Ser Ala Gly Ser Gly Ile Phe Pro Ser Ile Leu Ala Ala Ala Met Ala
610 615 620

Ala Ile Val Ala Ser Trp Thr Ile Leu
625 630

<210> 3
<211> 18
<212> PRT
<213> Aspergillus fumigatus

<400> 3

Asp Ser Ala Ser Tyr Tyr Lys Asp Tyr Ser Asn Ala Val Ser Gly Lys
1 5 10 15

Ala Asp

<210> 4
<211> 18
<212> PRT
<213> Aspergillus fumigatus

<220>
<221> MISC_FEATURE
<222> (13)..(13)
<223> unknown

<220>
<221> MISC_FEATURE
<222> (16)..(16)
<223> unknown

<400> 4

Ala Leu Pro Asn Ala Pro Asp Gly Tyr Thr Pro Ser Xaa Val Gly Xaa
1 5 10 15

Pro Ala

<210> 5
<211> 5
<212> PRT
<213> Aspergillus fumigatus

<400> 5

Asp Ser Ala Ser Tyr
1 5

<210> 6
<211> 10
<212> PRT
<213> Aspergillus fumigatus

<400> 6

Pro Val Val Val Ala Asp Gly Asn Tyr Pro
1 5 10

<210> 7
<211> 10
<212> PRT
<213> Aspergillus fumigatus

<220>
<221> MISC_FEATURE
<222> (1)..(1)
<223> unknown

<400> 7

Xaa Thr Ser Ser Thr Leu Phe Asn Gln Phe
1 5 10

<210> 8
<211> 19
<212> PRT
<213> Aspergillus fumigatus

<400> 8

Lys Asp Phe Phe Ser His Val Lys Ile Gln Asp Phe Asp Ala Val Gly
1 5 10 15

Tyr Ile Asp

<210> 9
<211> 5
<212> PRT
<213> Aspergillus fumigatus

<400> 9

Ala Leu Pro Asn Ala
1 5

<210> 10
<211> 14
<212> PRT

<213> Aspergillus fumigatus

<220>
<221> MISC_FEATURE
<222> (12)..(12)
<223> unknown

<400> 10

```
Asn Thr Ala Thr Ala Ile Lys Ala Phe Asp Ser Xaa Thr Pro
1               5                   10
```

<210> 11
<211> 39
<212> DNA
<213> Artificial Sequence

<220>
<223> Primer

<400> 11
gactcgagtc gacatcgatt tttttttttt tttttttv 39

<210> 12
<211> 26
<212> DNA
<213> Artificial Sequence

<220>
<223> Primer

<400> 12
gactttgacg ctgtggggta catcga 26

<210> 13
<211> 32
<212> DNA
<213> Artificial Sequence

<220>
<223> Primer

<400> 13
tacttgtgac gatggcgtta gttccgaaaa ct 32

<210> 14
<211> 18
<212> DNA
<213> Artificial Sequence

<220>
<223> Primer

<400> 14
ctttgcggca ctgcgaat 18

<210> 15

<211> 18
<212> DNA
<213> Artificial Sequence

<220>
<223> Primer

<400> 15
atatttgatc ttattgtc 18

<210> 16
<211> 26
<212> DNA
<213> Artificial Sequence

<220>
<223> Primer

<400> 16
ggcgcaggtg cgattaaggc ttttga 26

<210> 17
<211> 27
<212> DNA
<213> Artificial Sequence

<220>
<223> Primer

<400> 17
ttcgcgaaac tctcgaaatg aattcta 27

<210> 18
<211> 36
<212> DNA
<213> Artificial Sequence

<220>
<223> Primer

<400> 18
cgcggatccg tcatgaagtc catcgcagtg gcgtgc 36

<210> 19
<211> 26
<212> DNA
<213> Artificial Sequence

<220>
<223> Primer

<400> 19
ttgactagtt tgaaccacac ttcaag 26

<210> 20
<211> 23
<212> DNA
<213> Artificial Sequence

<220>
<223> Primer

<400> 20
gaattctggt gttttgatct ttt 23

<210> 21
<211> 36
<212> DNA
<213> Artificial Sequence

<220>
<223> Primer

<400> 21
agcaccgcta gcaccggaca ataataggcc ggcgac 36

<210> 22
<211> 48
<212> DNA
<213> Artificial Sequence

<220>
<223> Primer

<400> 22
tccggtgcta gcggtgctgc cctgcccaat gcccccgatg gatacaca 48

<210> 23
<211> 21
<212> DNA
<213> Artificial Sequence

<220>
<223> Primer

<400> 23
gaagtccttc atcgcagaag t 21

<210> 24
<211> 24
<212> DNA
<213> Artificial Sequence

<220>
<223> Primer

<400> 24
ctgatattta cgtaaaaatc gtca 24

<210> 25
<211> 27
<212> DNA
<213> Artificial Sequence

<220>
<223> Primer

<400> 25
cttgcctcga cgcgtctgaa gccatga 27